# EUROPEAN PATENT APPLICATION

(11) **EP 2 025 684 A1**
(43) Date of publication of application: **18.02.2009**
(21) Application number: 07016032.0
(22) Date of filing: 15.08.2007
(51) Int. Cl.: C07K 14/605, A61K 38/26

(54) **Glucagon analogues**

(71) Applicant: Zealand Pharma A/S, 2600 Glostrup (DK)
(72) Inventor: The designation of the inventor has not yet been filed
(74) Representative: Forrest, Graham Robert

(57) **Abstract**

The invention provides materials and methods for promoting weight loss or preventing weight gain in a subject, comprising administering to the subject a glucagon analogue peptide which has improved selectivity for the GLP-1 receptor as compared to human glucagon. The invention also provides novel glucagon analogue peptides having increased selectivity for the GLP-1 receptor as compared to human glucagon, and their use for the treatment of obesity, eating disorders, metabolic syndrome, and non-alcoholic liver steatosis.

## Description

### FIELD OF THE INVENTION

The present invention relates to glucagon analogues and their medical use, for example in the treatment of excess food intake, obesity and excess weight.

### BACKGROUND OF THE INVENTION

Treatment of obese patients with the peptide oxyntomodulin (Oxm) has been shown effective in obtaining weight loss, and several attempts have been made to obtain a useful medicament based on the native human oxyntomodulin sequence or modifications thereof [see, for example, WO03/022304 and WO2004/062685]. Oxyntomodulin is known to activate both the GLP-1 (glucagon-like peptide 1) receptor and the glucagon receptor, and it has been suggested that the GLP-1 receptor is essential for the anorectic function of Oxm.

Oxyntomodulin is derived from proglucagon, which is a 158 amino acid peptide processed differently in different organs. Whereas glucagon (29 amino acids in length; His-Ser-Gln-Gly-Thr-Phe-Thr-Ser-Asp-Tyr-Ser-Lys-Tyr-Leu-Asp-Ser-Arg-Arg-Ala-Gln-Asp-Phe-Val-Gln-Trp-Leu-Met-Asn-Thr) is produced in the pancreas, the longer 37 amino acid peptide oxyntomodulin (His-Ser-Gln-Gly-Thr-Phe-Thr-Ser-Asp-Tyr-Ser-Lys-Tyr-Leu-Asp-Ser-Arg-Arg-Ala-Gln-Asp-Phe-Val-Gln-Trp-Leu-Met-Asn-Thr-Lys-Arg-Asn-Arg-Asn-Asn-Ile-Ala) is produced in the intestine and brain. Oxyntomodulin consists of the full length glucagon and a C-terminal octapeptide (termed "intervening peptide 1" or IP-1, sequence Lys-Arg-Asn-Arg-Asn-Asn-Ile-Ala). Both glucagon and IP-1 fail to affect food intake when administered separately (Dakin, C.L. et al. (2001), Endocrin. 142, 4244-4250).

Both peptides are a substrate for the endogenous enzyme dipeptidyl peptidase which cleaves at the C-terminal side of Ser2 (Zhu, L. et al. (2003), J. Biol. Chem. 278, 22418-22423). Moreover it is found that a proteolytic fragment of glucagon is liberated following cleavage of glucagon at the Arg17-Arg18 amino acid doublet (Blache, P. at al. (1993) J. Biol. Chem. 268, 21748-21753).

### SUMMARY OF THE INVENTION

Without wishing to be bound by any particular theory, the present inventors believe that the anorectic effects of Oxm can be mimicked by analogues of glucagon which have an increased selectivity for the GLP-1 receptor. This enables a peptide to be used which has pharmacological effects similar to oxyntomodulin, but potentially has a shorter primary peptide chain like that of glucagon to facilitate production through solid phase peptide synthesis.

It is also believed that glucagon agonist activity may also be required for the anorectic effect to be demonstrated.

Thus, in its broadest form the invention provides a method of promoting weight loss or preventing weight gain in a subject, comprising administering to the subject a glucagon analogue peptide, said glucagon analogue peptide having improved selectivity for the GLP-1 receptor as compared to human glucagon. In some embodiments, the invention provides peptides and their use for the treatment of obesity, eating disorders, metabolic syndrome, and non-alcoholic liver steatosis.

Also provided is the use of a glucagon analogue peptide in the preparation of a medicament for promoting weight loss or preventing weight gain in a subject, the glucagon analogue peptide having higher GLP-1 receptor selectivity than human glucagon.

The methods and uses of the invention also extend to nucleic acids encoding the glucagon analogue peptides, expression vectors comprising such nucleic acids, and host cells containing such nucleic acids or expression vectors.

Also provided is a glucagon analogue peptide having higher GLP-1 receptor selectivity than human glucagon, or a nucleic acid, expression vector or host cell as described, for use in a method of promoting weight loss or preventing weight gain in a subject.

Also provided is a glucagon analogue peptide having higher GLP-1 receptor selectivity than human glucagon, or a nucleic acid, expression vector or host cell as described, for use in a method of medical treatment.

Typically the glucagon analogue peptide also has glucagon agonist activity, i.e. it is a GLP-1/glucagon co-agonist.

The GLP-1 or glucagon agonist activity of any given glucagon analogue peptide may be quantified by determining an EC50 value for that peptide in a selected assay for GLP-1 or glucagon activity. As the skilled person will be well aware, the EC50 value is a measure of the concentration at which half of that compound's maximal activity in the particular assay is achieved. In this specification, the EC50 value in an assay for GLP-1 or glucagon agonist activity will be referred to as EC50[GLP-1] and EC50[Glu] respectively. Where EC50 values for different compounds are compared, it will be understood that they describe the activity of the relevant compounds in the same assay under otherwise identical conditions.

The ratio EC50[Glu]/EC50[GLP-1] for the glucagon analogue peptide may be greater than the ratio EC50[Glu]/EC50[GLP-1] for glucagon. This may be interpreted to mean that the glucagon analogue peptide has a greater selectivity for GLP-1 receptor than glucagon.

Additionally or alternatively to having improved selectivity for the GLP-1 receptor, as described above, the glucagon analogue peptide may have greater activity in a lipogenesis assay than human glucagon.

The glucagon analogue peptide may comprise a substitution at one or more of positions 12, 16, 17, 18, 20, 23, 24, 27, 28 or 29 relative to human glucagon. Additionally or alternatively it may comprise a deletion at one or more of positions 27, 28 or 29 relative to human glucagon.

A substitution at position 12 may incorporate the residue Arg, His, Ser, Glu, Asp, or Ala.

A substitution at position 16 may incorporate the residue Thr, Lys, Arg, His, Glu, Asp, Asn, Ala or Gly.

A substitution at position 17 may incorporate the residue Lys, His, Glu, Asp, Gln, Ser, Leu or Ala.

A substitution at position 18 may incorporate the residue Lys, His, Glu, Asp, Gln, Tyr, Ser, Leu or Ala.

A substitution at position 20 may incorporate the residue Lys, Arg, His, Glu, Asp, Ser or Ala.

A substitution at position 23 may incorporate a hydrophobic residue, which may be Ile, Leu or Ala.

A substitution at position 24 may incorporate the residue Lys, Arg, His, Glu, Asp, Ser, Leu or Ala.

A substitution at position 27 may incorporate the residue Lys, Arg, His, Glu, Asp, Ser, Leu or Ala, or any other oxidatively stable Met-replacement amino acid as defined below

A substitution at position 28 may incorporate the residue Lys, Arg, His, Glu, Asp, Ser, Leu or Ala.

A substitution at position 29 may incorporate the residue Lys, Arg,, His, Glu, Asp, Ser, Ala, Gly or Leu.

Additionally or alternatively, the glucagon analogue peptide may comprise a deletion at one or more of positions 27, 28 and 29. If there is a deletion at position 28, there is preferably also a deletion at position 29. Similarly, if there is a deletion at position 27, there is preferably also a deletion at positions 28 and 29.

For example, the glucagon analogue peptide may have substitutions at position 12, position 20, positions 12 and 20, positions 12 and 24, or positions 12 and 29. It may have the specific residues Arg12, Ala20, Arg12/Lys20, Arg12/Lys24, or Arg12/Lys29. Such peptides have been described by DiMarchi et al. (19th American Peptide Symposium, 2005) and Levy et al. (listed as "Levy APS" on the website http://www.indiana.edu/∼rdmweb/?page=research . Such substitutions were shown to increase GLP-1 agonist activity. However the authors did not attribute any anorectic activity to their peptides. Any of these substitutions or combinations of substitutions may be combined with Leu27 if desired.

Thus examples of specific peptide sequences which may be used include:
HSQGTFTSDYSRYLDSRRAQDFVQWLMNT;
HSQGTFTSDYSRYLDSRRAKDFVQWLMNT;
HSQGTFTSDYSRYLDSRRAQDFVKWLMNT; and
HSQGTFTSDYSRYLDSRRAQDFVQWLMNK
as well as
HSQGTFTSDYSRYLDSRRAQDFVQWLLNT;
HSQGTFTSDYSRYLDSRRAKDFVQWLLNT;
HSQGTFTSDYSRYLDSRRAQDFVKWLLNT; and
HSQGTFTSDYSRYLDSRRAQDFVQWLLNK.

The glucagon analogue peptide may comprise substitutions at one or more of positions 16, 20, 24, 27 and 28. Optionally, it may also comprise substitutions at positions 12 and/or 17. For example it may have substitutions at positions 12, 16, 20, 24, 27 and 28, or at positions 16, 17, 20, 24, 27 and 28. For example, the specific substitutions may be one or more of Arg12, Arg16, Asp20, Ala24, Leu27 and/or Ser28. Alternatively, they may be one or more of Arg16, Ala17, Asp20, Ala24, Lys27 and/or Ser28. In some embodiments they may include the specific residues Arg12/Arg16,/Asp20,/Ala24/Leu27/Ser28. Alternatively they may include the specific residues Arg16/Ala17/Asp20/Ala24/Lys27/Ser28.

Further specific peptide sequences which may be used include:
HSQGTFTSDYSRYLDRRRADDFVAWLLST;
HSQGTFTSDYSKYLDRARADDFVAWLKST;
HSQGTFTSDYSKYLDRKVAEEFVAWLKKT;
HSQGTFTSDYSKYLDRRHADDFVAWLKST;
HSQGTFTSDYSKYLDERAAKEFVAWLKST;
HSQGTFTSDYSKYLDERAAEEFVAWLKST;
HSQGTFTSDYSKYLDEKRAEEFVAWLKST;
HSQGTFTSDYSKYLDEKRVEEFVAWLKST;
HSQGTFTSDYSKYLDERAAKEFVEWLKST;
HSQGTFTSDYSKYLDERAAKEFVKWLKST;
HSQGTFTSDYSKYLDERAAREFVAWLKST;
HSQGTFTSDYSKYLDRARAEEFVAWLKST;
HSQGTFTSDYSKYLDHARAAEFVAWLKAA;
HSQGTFTSDYSKYLDERRADDFVAWLKST;
HSQGTFTSDYSKYLDERAAEEFVKWLKST;
HSQGTFTSDYSKYLDEKKAEEFVAWLKST; and
HSQGTFTSDYSKYLDERRVEEFVAWLKST.

Any of these peptides may additionally comprise a Z¹ or Z² group as described below. For example, they may comprise an N terminal R¹-Z¹- or C-terminal -Z²-R² group. In particular, the Z¹ or Z² group may be Lys₃, Lys₄, Lys₅, Lys₆ or Lys₇. In some embodiments it may be preferred that the above peptides are conjugated at the C-terminus with Lys₃, Lys₄, Lys₅, Lys₆ or Lys₇. A pharmaceutically acceptable salt or derivative of any of the above peptides may also be used.

Without wishing to be bound by any particular theory, it is believed that substitutions (or deletion, as appropriate) at all of the positions listed above may be particularly useful for enhancing GLP-1 agonist activity and/or GLP-1 selectivity.

Substitution at various of these positions may have additional benefits. For example, substitution at position 27 may improve oxidative stability of the glucagon analogue peptide. Particular examples of substitution at position 27 may introduce the residue Lys, Glu, Ser or Leu.

Substitution at one or more of positions 20, 24 and 28 may improve resistance to deamidation. Particular examples of substitution at position 20 may introduce the residue Lys or Glu. Particular examples of substitution at position 24 may introduce the residue Lys, Glu or Ala. Particular examples of substitution at position 28 may introduce the residue Lys, Glu or Ser.

Thus the glucagon analogue peptide may have an increased shelf life when formulated as a medicament and/or have a longer duration of action in vivo.

Thus the glucagon analogue peptide may comprise a substitution at one or more of these positions, in combination with one or more changes (substitution or deletion) at one or more of the other positions listed above.

Residues 16, 20, 24, 27 and 28 are believed to contribute a hydrophilic face to an amphipathic alpha-helix. In certain embodiments it may be desirable to replace these amino acids with other polar or charged amino acids. Thus, a substitution at position 16 may incorporate the residue Glu or Ser. A substitution at position 20 may incorporate the residue Lys, Glu or Ser. A substitution at position 24 may incorporate the residue Lys, Glu or Ser. A substitution at position 27 may incorporate the residue Lys, Glu or Ser. A substitution at position 28 may incorporate the residue Lys, Glu or Ser.

Certain substitutions at the C-terminal end of the molecule may paticularly enhance GLP-1 agonist activity or selectivity. Thus, a substitution at position 23 may incorporate the residue Ile, Leu or Ala. A substitution at position 24 may incorporate the residue Lys, Glu, Ser or Leu. A substitution at position 27 may incorporate the residue Lys, Glu, Ser or Leu. A substitution at position 28 may incorporate the residue Lys, Glu or Ser. A substitution at position 29 may incorporate the residue Thr, Gly, Lys, Glu or Ser.

Certain mutations at other positions within the molecule may also enhance GLP-1 agonist activity or selectivity. Thus a substitution at position 12 may incorporate the residue Arg, Glu or Ser. A substitution at position 17 may incorporate the residue Lys, Glu, Ser, Leu or Ala. A substitution at position 18 may incorporate the residue Lys, Glu, Ser, Leu or Ala.

The invention relates specifically to peptides which are analogues of glucagon and does not extend to analogues of oxyntomodulin. Thus, typically, the glucagon analogue peptide has a pharmacologically active portion which is no greater than 29 amino acids in length. It does not possess a portion corresponding to the IP-1 portion of oxyntomodulin.

The pharmacologically active portion of the molecule may comprise one, two, three, four, five, six, seven, eight, nine or tensubstitutions relative to human glucagon. Thus it may have at least 65%, at least 70%, at least 75%, at least 80%, at least 85% or at least 90% sequence identity with human glucagon, and/or less than 97%, 94%, 90%, 87%, 83%, 80%, 76%, 73% or 69% identity with human glucagon.

However, the analogue may comprise an additional N- or C-terminal peptide sequence of 3-20 amino acids, for example to stabilise the conformation and/or secondary structure of the glucagon analogue peptide, and/or to make the glucagon analogue peptide more resistant to enzymatic hydrolysis, e.g. as described in WO99/46283. For consistency with the formulae shown below, these additional N- and C-terminal peptide sequences may be designated Z¹ and Z² respectively. When present at the C-terminus (Z²), this additional peptide sequence typically has no more than 25% sequence identity with the corresponding sequence of IP-1. It may be preferred that the peptides have no more than 80%, 75%, 70% or 65% identity with oxyntomodulin.

"Percent (%) amino acid sequence identity" with respect to the another polypeptide sequence (e.g. glucagon, Oxm or IP-1) is calculated as the percentage of amino acid residues in a glucagon analogue peptide sequence that are identical with corresponding amino acid residues in the corresponding sequence of that other polypeptide when the two are aligned with one another, introducing gaps for optimal alignment if necessary.

Preferred additional N- or C-terminal peptide sequences are 3 to 10, e.g. 4 to 7 amino acids in length. They may comprise or consist exclusively of amino acids selcted from Glu, Lys or Met. In certain embodiments they consist of three, four, five, six or seven consecutive lysine residues (i.e. Lys₃, Lys₄, Lys₅, Lys₆ or Lys₇).

Thus, within the constraints set out above, the glucagon analogue peptide may have the formula: 
R¹-Z¹-His-Ser-Gln-Gly-Thr-Phe-Thr-Ser-Asp-Tyr-Ser-X12-Tyr-Leu-Asp-X16-X17-X18-Ala-X20-Asp-
Phe-X23-X24-Trp-Leu-X27-X28-X29-Z²-R²
 wherein:
- R¹: is hydrogen, C₁₋₄ alkyl (e.g. methyl), acetyl, formyl, benzoyl or trifluoroacetyl
X12: Lys, Arg, His, Ser, Glu, Asp, Ala;
X16: Ser, Thr, Lys, Arg, His, Glu, Asp, Asn, Ala, Gly;
X17: Arg, Lys, His, Glu, Asp, Gln, Ala, Ser or Leu;
X18: Arg, Lys, His, Glu, Asp, Gln, Tyr, Ala, Ser or Leu;
X20: Gln, Lys, Arg, His, Glu, Asp, Ser, Ala;
X23: Val, Ile, Leu, Ala;
X24: Gln, Lys, Arg, His, Glu, Asp, Ser, Ala or Leu;
X27: Met, Lys, Arg, His, Glu, Asp, Ser, Leu, Ala or absent;
X28: Asn, Lys, Arg, His, Glu, Asp, Ser, Ala or absent;
X29: Thr, Lys, Arg, His, Glu, Asp, Ser, Ala, Gly, Leu or absent;
- R²: is NH₂ or OH;
Z¹ and Z² are independently absent or a peptide sequence of 1-20 amino acid units selected from the group consisting of Ala, Leu, Ser, Thr, Tyr, Asn, Gln, Asp, Glu, Lys, Arg, His, Met, Har, Dbu, Dpr and Orn;
or a pharmaceutically acceptable salt or derivative thereof.

Particular features of these compounds are as described above. For instance, the glucagon analogue peptide may be:
R¹-Z¹- HSQGTFTSDYSRYLDRRRADDFVAWLLST -Z²-R²;
R¹-Z¹- HSQGTFTSDYSKYLDRARADDFVAWLKST -Z²-R²;
R¹-Z¹-HSQGTFTSDYSKYLDRKVAEEFVAWLKKT-Z²-R²;
R¹-Z¹-HSQGTFTSDYSKYLDRRHADDFVAWLKST-Z²-R²;
R¹-Z¹-HSQGTFTSDYSKYLDERAAKEFVAWLKST-Z²-R²;
R¹-Z¹-HSQGTFTSDYSKYLDERAAEEFVAWLKST-Z²-R²;
R¹-Z¹-HSQGTFTSDYSKYLDEKRAEEFVAWLKST-Z²-R²;
R¹-Z¹-HSQGTFTSDYSKYLDEKRVEEFVAWLKST-Z²-R²;
R¹-Z¹-HSQGTFTSDYSKYLDERAAKEFVEWLKST-Z²-R²;
R¹-Z¹-HSQGTFTSDYSKYLDERAAKEFVKWLKST-Z²-R²;
R¹-Z¹-HSQGTFTSDYSKYLDERAAREFVAWLKST-Z²-R²;
R¹-Z¹-HSQGTFTSDYSKYLDRARAEEFVAWLKST-Z²-R²;
R¹-Z¹-HSQGTFTSDYSKYLDHARAAEFVAWLKAA-Z²-R²;
R¹-Z¹-HSQGTFTSDYSKYLDERRADDFVAWLKST-Z²-R²;
R¹-Z¹-HSQGTFTSDYSKYLDERAAEEFVKWLKST-Z²-R²;
R¹-Z¹-HSQGTFTSDYSKYLDEKKAEEFVAWLKST-Z²-R²; or
R¹-Z¹-HSQGTFTSDYSKYLDERRVEEFVAWLKST-Z²-R² or
R¹-Z¹-HSQGTFTSDYSRYLDSRRAQDFVQWLMNT-Z²-R²;
R¹-Z¹-HSQGTFTSDYSRYLDSRRAKDFVQWLMNT-Z²-R²;
R¹-Z¹-HSQGTFTSDYSRYLDSRRAQDFVKWLMNT-Z²-R²; or
R¹-Z¹-HSQGTFTSDYSRYLDSRRAQDFVQWLMNK-Z²-R² or
R¹-Z¹-HSQGTFTSDYSRYLDSRRAQDFVQWLLNT-Z²-R²;
R¹-Z¹-HSQGTFTSDYSRYLDSRRAKDFVQWLLNT-Z²-R²;
R¹-Z¹-HSQGTFTSDYSRYLDSRRAQDFVKWLLNT-Z²-R²; or
R¹-Z¹-HSQGTFTSDYSRYLDSRRAQDFVQWLLNK-Z²-R²;
or a pharmaceutically acceptable salt or derivative thereof.

In some embodiments, it may be preferred that the Z² group is Lys₃, Lys₄, Lys₅ Lys₆ or Lys₇. Optionally, Z¹ may be absent. Thus, in some embodiments the peptide may consist of the pharmacologically active portion C-terminally conjugated with Lys₃, Lys₄, Lys₅, Lys₆ or Lys₇.

In addition, the glucagon analogue peptide may have the formula: 
R¹-Z¹-His-Ser-Gln-Gly-Thr-Phe-Thr-Ser-Asp-Tyr-Ser-Lys-Tyr-Leu-Asp-X16-X17-X18-Ala-X20-Asp-
Phe-X23-X24-Trp-Leu-X27-X28-X29-Z²-R²
 wherein:
R¹ is hydrogen, C₁₋₄ alkyl (e.g. methyl), acetyl, formyl, benzoyl or trifluoroacetyl
X16: Ser, Thr, Lys, Arg, His;
X17: Ala, Ser or Leu;
X18: Arg, Lys, His, Ala, Ser or Leu;
X20: Gln, Lys, Arg, His, Glu, Asp, Ser, Ala;
X23: Val, Ile, Leu, Ala;
X24: Ser, Ala or Leu;
X27: Lys, Arg, His,;
X28:Ser, Ala;
X29: Thr, Lys, Arg, His, Glu, Asp, Ser, Ala, Gly, Leu or absent;
R² is NH₂ or OH;
Z¹ and Z² are independently absent or a peptide sequence of 1-10 amino acid units selected from the group consisting of Ala, Leu, Ser, Thr, Tyr, Asn, Gln, Asp, Glu, Lys, Arg, His, Met, Har, Dbu, Dpr and Orn;
or a pharmaceutically acceptable salt or derivative thereof.

In addition, the glucagon analogue peptide may have the formula: 
R1-Z1-His-Ser-Gln-Gly-Thr-Phe-Thr-Ser-Asp-Tyr-Ser-Lys-Tyr-Leu-Asp-X16-X17-X18-Ala-X20-Asp-
Phe-X23-X24-Trp-Leu-X27-X28-X29-Z2-R2
 wherein:
R¹ is hydrogen, C₁₋₄ alkyl (e.g. methyl), acetyl, formyl, benzoyl or trifluoroacetyl
X16: Ser, Lys, Arg, His, Glu;
X17: Ala, Arg, Lys, Glu, Leu;
X18: Arg, Lys, His, Ala, Ser or Leu;
X20: Gln, Lys, Arg, His, Glu, Asp, Ser, Ala, Leu;
X23: Val, Ile, Leu, Ala;
X24: Ala, Leu, Lys, Glu, Ser;
X27: Lys, Arg, His,;
X28:Ser, Lys, Glu;
X29: Thr, Lys, Glu, Ser, Ala, Gly, Leu or absent;
R² is NH₂ or OH; and
Z¹ and Z² are independently absent or a peptide sequence of 1-10 amino acid units selected from the group consisting of Ala, Leu, Ser, Thr, Tyr, Asn, Gln, Asp, Glu, Lys, Arg, His, Met, Har, Dbu, Dpr and Orn;
or a pharmaceutically acceptable salt or derivative thereof.
Particular features of these compounds are described above.

The invention also provides novel compounds with anorectic activity. These compounds of the invention may consequently be used in the methods described above.

Thus, the invention provides a compound of the formula: 
R¹-Z¹-His-Ser-Gln-Gly-Thr-Phe-Thr-Ser-Asp-Tyr-Ser-X12-Tyr-Leu-Asp-X16-X17-X18-Ala-X20-Asp-
Phe-X23-X24-Trp-Leu-X27-X28-X29-Z²-R²
 wherein:
R¹ is hydrogen, C₁₋₄ alkyl (e.g. methyl), acetyl, formyl, benzoyl or trifluoroacetyl
   X12: Lys, Arg, His, Ser, Glu, Asp, Ala;
   X16: Ser, Thr, Lys, Arg, His, Glu, Asp, Asn, Ala, Gly;
   X17: Arg, Lys, His, Glu, Asp, Gln, Ala, Ser or Leu;
   X18: Arg, Lys, His, Glu, Asp, Gln, Tyr, Ala, Ser or Leu;
   X20: Gln, Lys, Arg, His, Glu, Asp, Ser, Ala;
   X23: Val, Ile, Leu, Ala;
   X24: Gln, Lys, Arg, His, Glu, Asp, Ser, Ala or Leu;
   X27: Met, Lys, Arg, His, Glu, Asp, Ser, Leu, Ala or absent;
   X28: Asn, Lys, Arg, His, Glu, Asp, Ser, Ala or absent;
   X29: Thr, Lys, Arg, His, Glu, Asp, Ser, Ala, Gly, Leu or absent;
R² is NH₂ or OH;
Z¹ and Z² are independently absent or a peptide sequence of 1-20 amino acid units selected from the group consisting of Ala, Leu, Ser, Thr, Tyr, Asn, Gln, Asp, Glu, Lys, Arg, His, Met, Har, Dbu, Dpr and Orn;
wherein the glucagon analogue peptide has a substitution relative to human glucagon at one or more of positions 16, 17, 18 or 29, and/or a deletion at one or more of positions 27, 28 and 29;
or a pharmaceutically acceptable salt or derivative thereof;
wherein said glucagon analogue peptide has higher GLP-1 receptor selectivity than human glucagon or wherein said glucagon analogue peptide has greater activity in a lipogenesis assay than human glucagon.

It may be desirable to combine a change (substitution or deletion, as appropriate) at one or more of these positions with a substitution at one or both of positions 12 or 20 as described above.

For example, a substitution at position 12 may incorporate the residue Arg, Glu or Ser, especially Arg.

A substitution at position 20 may incorporate the residue Lys, Glu or Ser, especially Lys.

The invention also provides a compound of the formula: 
R1-Z1-His-Ser-Gln-Gly-Thr-Phe-Thr-Ser-Asp-Tyr-Ser-X12-Tyr-Leu-Asp-X16-X17-X18-Ala-X20-Asp-
Phe-X23-X24-Trp-Leu-X27-X28-X29-Z2-R2
 wherein:
R¹ is hydrogen, C₁₋₄ alkyl (e.g. methyl), acetyl, formyl, benzoyl or trifluoroacetyl
   X12: Lys, Arg, His, Ser, Glu, Asp, Ala;
   X16: Ser, Thr, Lys, Arg, His, Glu, Asp, Asn, Ala, Gly;
   X17: Arg, Lys, His, Glu, Asp, Gln, Ala, Ser or Leu;
   X18: Arg, Lys, His, Glu, Asp, Gln, Tyr, Ala, Ser or Leu;
   X20: Gln, Lys, Arg, His, Glu, Asp, Ser, Ala;
   X23: Val, Ile, Leu, Ala;
   X24: Gln, Lys, Arg, His, Glu, Asp, Ser, Ala or Leu;
   X27: Met, Lys, Arg, His, Glu, Asp, Ser, Leu, Ala or absent;
   X28: Asn, Lys, Arg, His, Glu, Asp, Ser, Ala or absent;
   X29: Thr, Lys, Arg, His, Glu, Asp, Ser, Ala, Gly, Leu or absent;
R² is NH₂ or OH;
Z¹ and Z² are independently absent or a peptide sequence of 1-20 amino acid units selected from the group consisting of Ala, Leu, Ser, Thr, Tyr, Asn, Gln, Asp, Glu, Lys, Arg, His, Met, Har, Dbu, Dpr and Orn;
wherein the glucagon analogue peptide has a substitution other than Ala at one or more of positions 23, 27 or 28 relative to human glucagon;
or a pharmaceutically acceptable salt or derivative thereof;
wherein said glucagon analogue peptide has higher GLP-1 receptor selectivity than human glucagon, or wherein said glucagon analogue peptide has greater activity in a lipogenesis assay than human glucagon.

It may be desirable to combine a substitution at one or more of these positions with a substitution at one or both of positions 12 or 20 as described above.

For example, a substitution at position 12 may incorporate the residue Arg, Glu or Ser, especially Arg.

A substitution at position 20 may incorporate the residue Lys, Glu or Ser, especially Lys.

Preferred substitutions at particular positions are as described above in relation to the first aspect of the invention.

Particularly preferred peptides may be:
R¹-Z¹-HSQGTFTSDYSRYLDRRRADDFVAWLLST -Z²-R²;
R¹-Z¹-HSQGTFTSDYSKYLDRARADDFVAWLKST-Z²-R²;
R¹-Z¹-HSQGTFTSDYSKYLDRKVAEEFVAWLKKT-Z²-R²;
R¹-Z¹-HSQGTFTSDYSKYLDRRHADDFVAWLKST-Z²-R²;
R¹-Z¹-HSQGTFTSDYSKYLDERAAKEFVAWLKST-Z²-R²;
R¹-Z¹-HSQGTFTSDYSKYLDERAAEEFVAWLKST-Z²-R²;
R¹-Z¹-HSQGTFTSDYSKYLDEKRAEEFVAWLKST-Z²-R²;
R¹-Z¹-HSQGTFTSDYSKYLDEKRVEEFVAWLKST-Z²-R²;
R¹-Z¹-HSQGTFTSDYSKYLDERAAKEFVEWLKST-Z²-R²;
R¹-Z¹-HSQGTFTSDYSKYLDERAAKEFVKWLKST-Z²-R²;
R¹-Z¹-HSQGTFTSDYSKYLDERAAREFVAWLKST-Z²-R²;
R¹-Z¹-HSQGTFTSDYSKYLDRARAEEFVAWLKST-Z²-R²;
R¹-Z¹-HSQGTFTSDYSKYLDHARAAEFVAWLKAA-Z²-R²;
R¹-Z¹-HSQGTFTSDYSKYLDERRADDFVAWLKST-Z²-R²;
R¹-Z¹-HSQGTFTSDYSKYLDERAAEEFVKWLKST-Z²-R²;
R¹-Z¹-HSQGTFTSDYSKYLDEKKAEEFVAWLKST-Z²-R²; or
R¹-Z¹-HSQGTFTSDYSKYLDERRVEEFVAWLKST-Z²-R²
or a pharmaceutically acceptable salt or derivative thereof.

Similarly, preferred features of Z¹ and Z² (when present) are as defined above. When Z² is present, it has no more than 25% sequence identity to the corresponding sequence of the IP-1 portion of Oxm.

In certain embodiments, when Z¹ is present R¹ may be H, and when Z² is present R² may be OH.

The invention further provides a nucleic acid encoding a glucagon analogue peptide of the invention, an expression vector comprising such a nucleic acid, and a host cell containing such a nucleic acid or expression vector.

As well as providing glucagon analogues that may have improved chemical stability and biological activity, the present invention also relates to providing compounds that have anti-obesity and/or feeding reducing activity in a mammal or human being in need of treatment.

As described above, it is believed that substitution at position 27 may improve oxidative stability. Substitution at one or more of positions 20, 24 and 28 may increase stability against deamidation. The glucagon analogue of the invention may therefore exhibit enhanced stability towards deamidation, and/or towards oxidative degradation, relative to the wild type glucagon as well as enhanced stability towards in vivo degradation.

In a further aspect, the present invention provides a composition comprising a glucagon analogue peptide as defined herein, or a salt or derivative thereof, a nucleic acid encoding such a glucagon analogue peptide, an expression vector comprising such a nucleic acid, or a host cell containing such a nucleic acid or expression vector, in admixture with a carrier. In preferred embodiments, the composition is a pharmaceutically acceptable composition and the carrier is a pharmaceutically acceptable carrier. The glucagon peptide analogue may be a pharmaceutically acceptable acid addition salt of the glucagon analogue.

The glucagon analogue peptides described find use in preventing weight gain or promoting weight loss. By "preventing" is meant inihibiting or reducing weight gain when compared to the absence of treatment, and is not necessarily meant to imply complete cessation of weight gain. Thus the peptides can be used for direct or indirect therapy of any condition caused or characterised by excess body weight, such as the treatment and/or prevention of obesity, and/or prevention of morbid obesity and metabolic syndrome, which is characterized by hypertriglyceridaemia, low HDL-cholesterol, elevated apolipoprotein B, small dense LDL particles, inflammatory profile, insulin resistance, hyperinsulinaemia, glucose intolerence, impaired fibrinolysis as well as endothelial and mitochondrial dysfunction. Metabolic syndrome is highly associated with increased prevalence of type-2 diabetes, hypertension, arteriosclerosis and coronary heart diseases. The peptides may cause a decrease in food intake and/or increased energy expenditure, resulting in the observed effect on body weight.

As already described, the invention extends to expression vectors comprising the above-described nucleic acid sequence, optionally in combination with sequences to direct its expression, and host cells transformed with the expression vectors. Preferably the host cells are capable of expressing and secreting the glucagon analogue. In a still further aspect, the present invention provides a method of producing the glucagon analogue, the method comprising culturing the host cells under conditions suitable for expressing the glucagon analogue and purifying the glucagon analogue thus produced.

The invention further provides a nucleic acid of the invention, an expression vector of the invention, or a host cell capable of expressing and secreting a glucagon analogue of the invention, for use in therapy. It will be understood that the nucleic acid, expression vector and host cells may be used for treatment of any of the disorders described herein which may be treated with the glucagon analogues themselves. References to a therapeutic composition comprising a glucagon analogue of the invention, or administration of a glucagon analogue of the invention, should therefore be construed to encompass administration of a nucleic acid, expression vector or host cell of the invention except where the context demands otherwise.

### DESCRIPTION OF THE FIGURES

Figure 1 shows body weight gain in high fat fed (DIO) mice treated s.c. with vehicle, Exendin-4 amide (1-39) (ZP24, H-HGEGTFTSDLSKQMEEEAVRLFIEWLKNGGPSSGAPPPS-NH₂), oxyntomodulin (ZP2232, H-HSQGTFTSDYSKYLDSRRAQDFVQWLMNTKRNRNNIA-OH) and the peptide ZP 2495 (H-HSQGTFTSDYSKYLDRARADDFVAWLKST-NH₂) for 28 days. Animals eating chow are also included for comparison.

Figure 2 shows that the weight-reducing effect of ZP2495 is sustained over time comparable to oxyntomodulin treatment and significantly different from exendin-4 treatment. Control peptides are the same as in Fig. 1.

### DETAILED DESCRIPTION OF THE INVENTION

Throughout the description and claims the conventional one-letter and three-letter codes for natural amino acids are used as well as generally accepted three letter codes for other α-amino acids, such as sarcosin (Sar), norleucine (Nle) and α-aminoisobutyric acid (Aib). All amino acid residues in peptides of the invention are preferably of the L-configuration. However, D-configuration amino acids may also be present.

The glucagon analogues of the present invention have one or more amino acid substitutions or deletions compared with native glucagon and as defined above. This definition also includes the synonym terms glucagon mimetics and/or glucagon agonists. Further, the analogue of the present invention may additionally have chemical modification of one or more of its amino acid side groups, α-carbon atoms, terminal amino group, or terminal carboxylic acid group. A chemical modification includes, but is not limited to, adding chemical moieties, creating new bonds, and removing chemical moieties. Modifications at amino acid side groups include, without limitation, acylation of lysine ε-amino groups, N-alkylation of arginine, histidine, or lysine, alkylation of glutamic or aspartic carboxylic acid groups, and deamidation of glutamine or asparagine. Modifications of the terminal amino include, without limitation, the des-amino, N-lower alkyl, N-di-lower alkyl, and N-acyl modifications. Modifications of the terminal carboxy group include, without limitation, the amide, lower alkyl amide, dialkyl amide, and lower alkyl ester modifications. Preferably herein lower alkyl is C₁-C₄ alkyl. Furthermore, one or more side groups, or terminal groups, may be protected by protective groups known to the ordinarily-skilled peptide chemist. The α-carbon of an amino acid may be mono- or di-methylated.

Where they are present, oxidatively stable Met-replacement amino acid means one which is selected among the group consisting of Met(O) (methionine sulfoxide), Met(O)₂ (methionine sulfone), Val, Ile, Lys, Asn, Glx (Glu or Gln), Tyr, Phe, Trp and preferably Leu, Lys, Nle, Ala, Ser, and Gly.

It should be understood that the peptides of the invention might also be provided in the form of a salt or other derivative. Salts include pharmaceutically acceptable salts such as acid addition salts and basic salts. Examples of acid addition salts include hydrochloride salts, citrate salts and acetate salts. Examples of basic salts include salts where the cation is selected from alkali metals, such as sodium and potassium, alkaline earth metals, such as calcium, and ammonium ions ⁺N(R³)₃(R⁴), where R³ and R⁴ independently designates optionally substituted C₁₋₆-alkyl, optionally substituted C₂₋₆-alkenyl, optionally substituted aryl, or optionally substituted heteroaryl. Other examples of pharmaceutically acceptable salts are described in "Remington's Pharmaceutical Sciences" ,17th edition. Ed. Alfonso R. Gennaro (Ed.), Mark Publishing Company, Easton, PA, U.S.A., 1985 and more recent editions, and in the Encyclopaedia of Pharmaceutical Technology.

Other derivatives of the glucagon analogues of the invention include coordination complexes with metal ions such as Mn²⁺ and Zn²⁺, esters such as *in vivo* hydrolysable esters, free acids or bases, hydrates, prodrugs or lipids. Esters can be formed between hydroxyl or carboxylic acid groups present in the compound and an appropriate carboxylic acid or alcohol reaction partner, using techniques well known in the art. Derivatives which as prodrugs of the compounds are convertible *in vivo* or *in vitro* into one of the parent compounds. Typically, at least one of the biological activities of compound will be reduced in the prodrug form of the compound, and can be activated by conversion of the prodrug to release the compound or a metabolite of it. Examples of prodrugs include the use of protecting groups which may be removed *in situ* releasing active compound or serve to inhibit clearance of the drug *in vivo.*

When present, Z¹ and Z² each independently represent a peptide sequence of 3-20 or 4-20 amino acid residues, e.g. in the range of 4-15, more preferably in the range of 4-10 in particular in the range of 4-7 amino acid residues, e.g., of 4, 5, 6 or 7 amino acid residues, such as 6 amino acid residues. Each of the amino acid residues in the peptide sequences Z may independently be selected from Ala, Leu, Ser, Thr, Tyr, Asn, Gln, Asp, Glu, Lys, Arg, His, Met, Orn. Preferably, the amino acid residues are selected from Ser, Thr, Tyr, Asn, Gln, Asp, Glu, Lys, Arg, His, Orn, and Met, as well as amino acids falling within formula I as defined in WO01/04156, e.g., Dbu (2,4 diaminobutyric acid) or Dpr (2,3-diaminopropanoic acid), more preferably from Glu, Lys, and Met, especially Lys. The above-mentioned amino acids may have either D- or L-configuration, but preferably the above-mentioned amino acids have an L-configuration. Particularly preferred sequences Z are sequences of four, five or six consecutive lysine residues, and particularly five or six consecutive lysine residues. Exemplary sequences Z are shown in WO 01/04156.

In certain preferred embodiments, Z¹ is absent. In such cases, Z² may be either present or absent.

### Agonist activity and receptor selectivity

The glucagon analogue peptides described in this specification may have higher GLP-1 receptor selectivity than human glucagon. In some embodiments, they may also have higher GLP-1 agonist activity than human glucagon.

Binding of the relevant compounds to GLP-1 or glucagon receptors may be used as an indication of agonist activity, but in general it is preferred to use a biological assay which measures intracellular signalling caused by binding of the compound to the relevant receptor. For example, production of cyclic AMP (cAMP) is often used to monitor both GLP-1 and glucagon receptor activity, and hence can be used to determine glucagon analogue peptide agonist activity towards each type of receptor.

The skilled person will be aware of suitable assay fomats, and examples are provided below. The GLP-1 receptor and/or the glucagon receptor may have the sequence of the receptors as described in the examples. E.g., the assays may make use the human glucagon receptor (Glucagon-R) having primary accession number P47871 (GI 1346144) and/or the human glucagon-like peptide 1 receptor (GLP-1 R) having primary accession number P43220 (GI 1169956). (Where sequences of precursor proteins are referred to, it should of course be understood that assays may make use of the mature protein, lacking the signal sequence).

EC50 values may be used as a numerical measure of agonist activity. An EC50 value is a measure of the concentration of a compound requried to achieve half of that compound's maximal activity in a particular assay. Thus a compound having EC50[GLP-1] lower than the EC50[GLP-1] of glucagon in a particular assay may be considered to have a higher GLP-1 agonist activity than glucagon.

By "higher selectivity" for GLP-1 is meant that the ratio of the compound's GLP-1 agonist activity to its glucagon agonist activity is higher than that of glucagon. That is to say, for a particular level of glucagon agonist activity, the analogue will display a higher level of GLP-1 agonist activity than glucagon. Since the EC50 values are inversely related to activity, this means that the ratio EC50[Glu]/EC50[GLP-1] for the glucagon analogue peptide may be greater than the ratio EC50[Glu]/EC50[GLP-1] for glucagon.

It may in some embodiments be preferred that the glucogon analogue has a higher GLP-1 agonist activity than human glucagon. (Accordingly, it may have a lower EC50[GLP-1] than the EC50[GLP-1] for human glucagon.)

However, in other embodiments, the GLP-1 agonist activity may be substantially similar to, or lower than, human glucagon, provided that the ratio of GLP-1/glucagon agonist activity remains higher for the analogue than for human glucagon (i.e., the glucagon agonist activity is correspondingly reduced).

As mentioned above, typically the glucagon analogue peptide also has glucagon agonist activity, i.e. it is a GLP-1/glucagon co-agonist.

The glucagon analogue peptide may have substantially similar glucagon agonist activity to human glucagon (indeed, it may have a higher activity provided that the GLP-1 agonist activity is correspondingly increased). However the glucagon agonist activity may also be lower than that of human glucagon, as long as it is sufficient for anorectic activity.

### Stability studies

The skilled person will be able to design appropriate methods (e.g. quantitative methods) for detection of degradation products of glucagon analogues, e.g. based on those described below. Degradation may occur as oxidation, hydrolysis and deamidation, depending on the identity and position of the amino acids in any given glucagon analogue, and conditions as pH, solution and temperature. The compounds can be ranked according to chemical stability, when the compounds are incubated under stressed conditions (i.e. conditions likely to cause degradation) and subsequently analysed for content of remaining intact peptide. In addition, the knowledge gained about major degradation products obtained under stressed conditions will be important for any later analytical method development.

### Quantitative Assays to detect glucagon analogues

The skilled person will also be capable of designing methods (e.g. quantitative methods) for detection of glucagon analogues in complex environments or solutions (e.g. plasma, urine, tissue homogenates, cell homogenates, saliva or similar) to investigate the absorption, distribution, metabolism and excretion of the glucagon analogues after administration to mammals or as part of functional studies of *in vitro* cell systems.

In one embodiment, a quantitative assay can be based on antibodies raised against the glucagon analogues or fragments thereof. The antibodies obtained from the immunized animals can be used for quantitative assays. In one example a direct sandwich ELISA can be prepared using a first antibody with affinity of one part of the molecule immobilized in a multi-well plate. The sample is then applied to the wells and the glucagon analogue is captured by the first antibody. The captured glucagon analogue is then recognized by a second antibody with affinity for another part of the glucagon analogue. The second antibody can be labeled with an enzyme (horseradish peroxidase, alkaline phosphatase or beta-galactosidase) or a radioisotope. The amount of captured glucagon analogue can then be detected by addition of a colorimetric substrate or direct counting of radio-emission or by scintillation. Alternatively, the amount of captured glucagon analogue can be detected indirectly by addition of a labeled antibody with affinity for the second antibody. The concentration in the sample can be estimated from the response obtained from an external standard curve containing known amounts of glucagon analogue. Alternatively, the antibodies can be used to prepare a direct competitive immuno assay, where an antibody specific for the glucagon analogue is immobilized on a multi-well plate and the sample incubated in the wells with a predefined fixed concentration of labeled glucagon analogue. The label can be an enzyme, a fluorophore, a radioisotope or biotin and detected using, for example, substrates (e.g. colorimetric, fluorometric or chemiluminiscent) specific for the enzymes, scintillation or avidin linked to an enzyme followed by detection as described above. The amount of bound labeled glucagon analogue can be detected by an appropriate method and the concentration of glucagon analogue present in the sample derived from the response obtained from an external standard curve as described above.

In another embodiment, a quantitative assay can be based on liquid chromatography tandem mass spectroscopy methodology. In such a set up, the response from a fragment specific for the glucagon analogue to be studied is monitored upon fragmentation of the parent compound induced by collision with an inert gas (He or Ar). Prior to fragmentation the sample components can be separated by reversed phase chromatography or the sample can be injected directly in the mass spectrometer. If suitable the sample can be subjected to pretreatment (i.e., addition of protease inhibitors, protein precipitation, solid phase extraction, immuno-affinity extraction, etc. The concentration of glucagon analogue present in the sample derived from the response obtained from an external standard curve as described above, potentially aftyer correction of the response using an internal standard similar to the glucagon analogue to be studied.

### Generation of specific antibodies

Specific antibodies against the glucagon analogues or fragments thereof can be induced in mammals and purified from the serum. The glucagon analogues or fragments can either be used directly with an adjuvant to immunize rabbits, mice or other mammals, or the glucagon analogues or fragments thereof can be chemically linked to a carrier molecule (i.e., keyhole limpet hemocyanin, ovalbumin, albumin etc.) and injected with an adjuvant. The injections can be repeated with 2-4 weeks intervals for extended periods to improve the affinity and selectivity of the antibodies. Polyclonal antibodies can be harvested directly from the serum. To obtain monoclonal antibodies, B cells isolated from immunized animals, preferably mice, should be fused with tumor cells to form antibody producing hybridomas. Screening and selection of the appropriate clones and antibodies can be performed using either immobilized glucagon analogues or peptides thereof followed by detection with labeled anti-antibodies. Alternatively the screening and selection could be based on immobilized antibodies followed by detection with labeled glucagon analogues or fragments thereof. In all cases, the label could be a radioisotope, an enzyme, a fluorophore or biotin and detected using, for example, substrates (e.g. colorimetric, fluorometric or chemiluminiscent) specific for the enzymes, scintillation or avidin linked to an enzyme followed by detection as described.

### Synthesis of glucagon analogues

It is preferred to synthesize the analogues of the invention by means of solid phase or liquid phase peptide synthesis. In this context, reference is given to WO 98/11125 and, amongst many others, Fields, GB et al., 2002, "Principles and practice of solid-phase peptide synthesis". In: Synthetic Peptides (2nd Edition) and the Examples herein.

Thus the glucagon analogues may be synthesized in a number of ways including for example, a method which comprises:
(a) synthesizing the peptide by means of solid phase or liquid phase peptide synthesis and recovering the synthetic peptide thus obtained; or
(b) when the peptide is constituted by naturally occurring amino acids, expressing a nucleic acid construct that encodes the peptide in a host cell and recovering the expression product from the host cell culture; or
(c) when the peptide is constituted by naturally occurring amino acids, effecting cell-free in vitro expression of a nucleic acid construct that encodes the peptide and recovering the expression product; or
a combination of methods of (a), (b), and (c) to obtain fragments of the peptide, subsequently ligating the fragments to obtain the peptide, and recovering the peptide.

Thus, for some analogues of the invention it may be advantageous to exploit genetic engineering techniques. This may be the case when the peptide is sufficiently large (or produced as a fusion construct) and when the peptide only includes naturally occurring amino acids that can be translated from RNA in living organisms.

For the purpose of recombinant gene technology nucleic acid fragments encoding the peptides of the invention are important chemical products. Hence, a further aspect of the present invention provides a nucleic acid molecule comprising a nucleic acid sequence encoding a glucagon analogue of the invention, where the peptide preferably is comprised by naturally occurring amino acids. The nucleic acid fragments of the invention are either DNA or RNA fragments.

The nucleic acid fragments of the invention will normally be inserted in suitable vectors to form cloning or expression vectors carrying the nucleic acid fragments of the invention; such novel vectors are also part of the invention. Details concerning the construction of these vectors of the invention will be discussed in context of transformed cells and microorganisms below. The vectors can, depending on purpose and type of application, be in the form of plasmids, phages, cosmids, mini-chromosomes, or virus, but also naked DNA which is only expressed transiently in certain cells is an important vector. Preferred cloning and expression vectors (plasmid vectors) of the invention are capable of autonomous replication, thereby enabling high copy-numbers for the purposes of high-level expression or high-level replication for subsequent cloning.

The general outline of a vector of the invention comprises the following features in the 5'→3' direction and in operable linkage: a promoter for driving expression of the nucleic acid fragment of the invention, optionally a nucleic acid sequence encoding a leader peptide enabling secretion (to the extracellular phase or, where applicable, into the periplasma) of or a leader peptide for multiple use e.g. combined secretion, purification tag and enzymatic trimming to correct peptide or integration into the membrane of the polypeptide fragment, the nucleic acid fragment encoding the peptide of the invention, and optionally a nucleic acid sequence encoding a terminator. When operating with expression vectors in producer strains or cell-lines it is for the purposes of genetic stability of the transformed cell preferred that the vector when introduced into a host cell is integrated in the host cell genome.

The vectors of the invention are used to transform host cells to produce the modified peptide of the invention. Such transformed cells, which are also part of the invention, can be cultured cells or cell lines used for propagation of the nucleic acid fragments and vectors of the invention, or used for recombinant production of the peptides of the invention.

Preferred transformed cells of the invention are micro-organisms such as bacteria (such as the species Escherichia (e.g. E. coli), Bacillus (e.g. Bacillus subtilis), Salmonella, or Mycobacterium (preferably non-pathogenic, e.g. M. bovis BCG), yeasts (such as Saccharomyces cerevisiae), and protozoans. Alternatively, the transformed cells are derived from a multicellular organism, i.e. it may be fungal cell, an insect cell, a plant cell, or a mammalian cell. Also cells derived from a human being are interesting, cf. the discussion of cell lines and vectors below. For the purposes of cloning and/or optimised expression it is preferred that the transformed cell is capable of replicating the nucleic acid fragment of the invention. Cells expressing the nucleic fragment are useful embodiments of the invention; they can be used for small-scale or large-scale preparation of the peptides of the invention.

When producing the peptide of the invention by means of transformed cells, it is convenient, although far from essential, that the expression product is either exported out into the culture medium or carried on the surface of the transformed cell.

When an effective producer cell has been identified it is preferred, on the basis thereof, to establish a stable cell line which carries the vector of the invention and which expresses the nucleic acid fragment encoding the peptide. Preferably, this stable cell line secretes or carries the peptide of the invention, thereby facilitating purification thereof.

In general, plasmid vectors containing replicon and control sequences, which are derived from species compatible with the host cell, are used in connection with the hosts. The vector ordinarily carries a replication site, as well as marking sequences, which are capable of providing phenotypic selection in transformed cells. For example, E. coli is typically transformed using pBR322 (but numerous other useful plasmids exist) a plasmid derived from an E. coli species (see, e.g., Bolivar et al., 1977). The pBR322 plasmid contains genes for ampicillin and tetracycline resistance and thus provides easy means for identifying transformed cells. The pBR plasmid, or other microbial plasmid or phage must also contain, or be modified to contain promoters, which can be used by the prokaryotic microorganism for expression.

Those promoters most commonly used in prokaryotic recombinant DNA construction include the β-lactamase (penicillinase) and lactose promoter systems (Chang et al., 1978; Itakura et al., 1977; Goeddel et al., 1979) and a tryptophan (trp) promoter system (Goeddel et al., 1979; EP 0 036 776 A). While these are the most commonly used, other microbial promoters have been discovered and utilized, and details concerning their nucleotide sequences have been published, enabling a skilled worker to ligate them functionally with plasmid vectors (Siebwenlist et al., 1980).

In addition to prokaryotes, eukaryotic microbes, such as yeast cultures may also be used, and also here the promoter should be capable of driving expression. Saccharomyces cerevisiae, or common baker's yeast, is the most commonly used among eukaryotic microorganisms, although a number of other strains are commonly available. For expression in Saccharomyces, the plasmid YRp7, for example, is commonly used (Stinchcomb et al., 1979; Kingsman et al., 1979; Tschemper et al., 1980). This plasmid already contains the trpl gene which provides a selection marker for a mutant strain of yeast lacking the ability to grow in tryptophan for example ATCC No. 44076 or PEP4-1 (Jones, 1977). The presence of the trpl lesion as a characteristic of the yeast host cell genome then provides an effective environment for detecting transformation by growth in the absence of tryptophan.

Suitable promoting sequences in yeast vectors include the promoters for 3-phosphoglycerate kinase (Hitzman et al., 1980) or other glycolytic enzymes (Hess et al., 1968; Holland et al., 1978), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. In constructing suitable expression plasmids, the termination sequences associated with these genes are also ligated into the expression vector 3' of the sequence desired to be expressed to provide polyadenylation of the mRNA and termination.

Other promoters, which have the additional advantage of transcription controlled by growth conditions are the promoter region for alcohol dehydrogenase 2, isocytochrome C, acid phosphatase, degradative enzymes associated with nitrogen metabolism, and the aforementioned glyceraldehyde-3-phosphate dehydrogenase, and enzymes responsible for maltose and galactose utilization. Any plasmid vector containing a yeast-compatible promoter, origin of replication and termination sequences is suitable.

In addition to microorganisms, cultures of cells derived from multicellular organisms may also be used as hosts. In principle, any such cell culture is workable, whether from vertebrate or invertebrate culture. However, interest has been greatest in vertebrate cells, and propagation of vertebrate in culture (tissue culture) has become a routine procedure in recent years (Tissue Culture, 1973). Examples of such useful host cell lines are VERO and HeLa cells, Chinese hamster ovary (CHO) cell lines, and W138, BHK, COS-7 293, Spodoptera frugiperda (SF) cells (commercially available as complete expression systems from i.a. Protein Sciences, 1000 Research Parkway, Meriden, CT 06450, U.S.A. and from Invitrogen), the D. melanogaster cell line S₂ available from Invitrogen, PO Box 2312, 9704 CH Groningen, The Netherlands, and MDCK cell lines.

Expression vectors for such cells ordinarily include (if necessary) an origin of replication, a promoter located in front of the gene to be expressed, along with any necessary ribosome binding sites, RNA splice sites, polyadenylation site, and transcriptional terminator sequences.

For use in mammalian cells, the control functions on the expression vectors are often provided by viral material. For example, commonly used promoters are derived from polyoma, Adenovirus 2, and most frequently Simian Virus 40 (SV40). The early and late promoters of SV40 virus are particularly useful because both are obtained easily from the virus as a fragment, which also contains the SV40 viral origin of replication (Fiers et al., 1978). Smaller or larger SV40 fragments may also be used, provided there is included the approximately 250 bp sequence extending from the Hindlll site toward the BgII site located in the viral origin of replication. Further, it is also possible, and often desirable, to utilize promoter or control sequences normally associated with the desired gene sequence, provided such control sequences are compatible with the host cell systems.

An origin of replication may be provided either by construction of the vector to include an exogenous origin, such as may be derived from SV40 or other viral (e.g. Polyoma, Adeno, VSV, BPV) or may be provided by the host cell chromosomal replication mechanism. If the vector is integrated into the host cell chromosome, the latter is often sufficient.

In order to obtain satisfactory yields in a recombinant production process, it may be advantageous to prepare the analogues as fusion proteins, either by fusing the peptide to a fusion partner that can serve as an affinity tag (for ease of purification) and/or by having multiple repeats of the peptide. These methods require presence of a suitable cleavage site for a peptidase, but the skilled person will know how to tailor the underlying genetic constructs.

After recombinant preparation, the peptides of the invention can be purified by methods generally known in the art, including multi-step chromatography (ion-exchange, size-exclusion, and affinity chromatographic techniques).

Alternatively, peptides comprised of naturally occurring amino acids can be prepared in vitro in cell free systems. This is especially expedient in cases where the peptides could be toxic for putative host cells. Thus, the present invention also contemplates use of cell-free in vitro translation/expression in order to prepare the peptides of the invention. In this context, reference is made to commercially available in vitro translation kits, materials, and technical documentation from e.g. Ambion Inc., 2130 Woodward, Austin, TX 78744-1832, USA.

Finally, the available methods can of course be combined so as to prepare e.g. semi-synthetic analogues. In such a set up, peptide fragments are prepared using at least 2 separate steps or methods, followed by ligation of the fragments to obtain the final peptide product.

### Pharmaceutical Compositions and Administration

The glucagon analogues of the present invention, or salts or derivatives thereof, may be formulated as pharmaceutical compositions prepared for storage or administration, and which comprise a therapeutically effective amount of a glucagon peptide of the present invention, or a salt or derivative thereof, in a pharmaceutically acceptable carrier.

The therapeutically effective amount of a compound of the present invention will depend on the route of administration, the type of mammal being treated, and the physical characteristics of the specific mammal under consideration. These factors and their relationship to determining this amount are well known to skilled practitioners in the medical arts. This amount and the method of administration can be tailored to achieve optimal efficacy so as to deliver the peptide to the large intestine, but will depend on such factors as weight, diet, concurrent medication and other factors, well known those skilled in the medical arts.

It is within the invention to provide a pharmaceutical composition, wherein the glucagon analogue, or a salt thereof is present in an amount effective to treat or prevent stomach and bowel-related disorders.

Pharmaceutically acceptable salts of the compounds of the invention having an acidic moiety can be formed using organic and inorganic bases. Suitable salts formed with bases include metal salts, such as alkali metal or alkaline earth metal salts, for example sodium, potassium, or magnesium salts; ammonia salts and organic amine salts, such as those formed with morpholine, thiomorpholine, piperidine, pyrrolidine, a mono-, di- or tri-lower alkylamine (e:g., ethyl-tert-butyl-, diethyl-, diisopropyl-, triethyl-, tributyl- or dimethylpropylamine), or a mono-, di- or trihydroxy lower alkylamine (e.g., mono-, di- or triethanolamine). Internal salts also may be formed. Similarly, when a compound of the present invention contains a basic moiety, salts can be formed using organic and inorganic acids. For example, salts can be formed from the following acids: acetic, propionic, lactic, citric, tartaric, succinic, fumaric, maleic, malonic, mandelic, malic, phthalic, hydrochloric, hydrobromic, phosphoric, nitric, sulfuric, methanesulfonic, napthalenesulfonic, benzenesulfonic, toluenesulfonic, and camphorsulfonic as well as other known pharmaceutically acceptable acids. Amino acid addition salts can also be formed with amino acids such as lysine, glycine, or phenylalanine.

As is apparent to one skilled in the medical art, a "therapeutically effective amount" of the peptides or pharmaceutical compositions of the present invention will vary depending upon the age, weight and mammalian species treated, the particular compounds employed, the particular mode of administration and the desired effects and the therapeutic indication. Because these factors and their relationship to determining this amount are well known in the medical arts, the determination of therapeutically effective dosage levels, the amount necessary to achieve the desired result of preventing and/or treating the intestine and stomach related diseases described herein, as well as other medical indications disclosed herein, will be within the ambit of the skilled person.

As used herein, "a therapeutically effective amount" is one which reduces symptoms of a given condition or pathology, and preferably which normalizes physiological responses in an individual with the condition or pathology. Reduction of symptoms or normalization of physiological responses can be determined using methods routine in the art and may vary with a given condition or pathology. In one aspect, a therapeutically effective amount of one or more glucagon analogues or pharmaceutical composition comprising the one or more glucagon analogues is an amount which restores a measurable physiological parameter to substantially the same value (preferably to within + 30%, more preferably to within + 20%, and still more preferably, to within 10% of the value) of the parameter in an individual without the condition or pathology.

In one embodiment of the invention administration of the compounds or pharmaceutical composition of the present invention is commenced at lower dosage levels, with dosage levels being increased until the desired effect of preventing/treating the relevant medical indication, such as intestine and stomach related diseases is achieved. This would define a therapeutically effective amount. For the peptides of the present invention, alone or as part of a pharmaceutical composition, such doses may be between about 0.01 mg/kg and 100 mg/kg body weight, such as between about 0.01 mg/kg and 10 mg/kg body weight, for example between 10-100 µg/kg body weight.

For therapeutic use, the chosen glucagon analogue is formulated with a carrier that is pharmaceutically acceptable and is appropriate for delivering the peptide by the chosen route of administration. For the purpose of the present invention, peripheral parenteral routes include intravenous, intramuscular, subcutaneous, and intra peritoneal routes of administration. Certain compounds used in the present invention may also be amenable to administration by the oral, rectal, nasal, or lower respiratory routes. These are so-called non-parenteral routes. The present pharmaceutical composition comprises a glucagon analogue of the invention, or a salt or derivative thereof and a pharmaceutically acceptable carrier. Suitable pharmaceutically acceptable carriers are those used conventionally with peptide-based drugs, such as diluents, excipients and the like. Pharmaceutically acceptable carriers for therapeutic use are well known in the pharmaceutical art, and are described, for example, in Remington's Pharmaceutical Sciences, Mack Publishing Co. (A. R. Gennaro edit. 1985). For example, sterile saline and phosphate-buffered saline at slightly acidic or physiological pH may be used. pH buffering agents may be phosphate, citrate, acetate, tris/hydroxymethyl)aminomethane (TRIS), N-Tris(hydroxymethyl)methyl -3- aminopropanesulphonic acid (TAPS), ammonium bicarbonate, diethanolamine, histidine, which is a preferred buffer, arginine, lysine, or acetate or mixtures thereof. Preferred buffer ranges are pH 4-8, pH 6.5-8, more preferably pH 7-7.5. Preservatives, such as para, meta, and ortho-cresol, methyl- and propylparaben, phenol, benzyl alcohol, sodium benzoate, benzoic acid, benzyl-benzoate, sorbic acid, propanoic acid, esters of p-hydroxybenzoic acid may be provided in the pharmaceutical composition. Stabilizers, preventing oxidation, deamidation, isomerisation, racemisation, cyclisation, peptide hydrolysis, such as e.g. ascorbic acid, methionine, tryptophane, EDTA, asparagine, lysine, arginine, glutamine and glycine may be provided in the pharmaceutical composition. Stabilizers, preventing aggregation, fibrillation and precipitation, such as Sodium dodecyl sulphate, polyethylene glycol, carboxymethyl cellulose, cyclodextrine may be provided in the pharmaceutical composition. Organic modifiers for solubilization or preventing aggregation, such as ethanol, acetic acid or acetate and salts thereof may be provided in the pharmaceutical composition. Isotonicity makers such as salts e.g. sodium chloride or most preferred carbohydrates e.g. dextrose, mannitol, lactose, trehalose, sucrose or mixtures thereof may be provided in the pharmaceutical composition.

Detergents, such as Tween 20, Tween 80, SDS, Poloxamers e.g. Pluronic F-68, Pluronic F-127, may be provided in the pharmaceutical composition. Dyes and even flavoring agents may be provided in the pharmaceutical composition. In another embodiment, a pharmaceutically acceptable acid addition salt of the glucagon peptide analogue is provided for. Suspending agents may be used.

Organic modifiers, such as ethanol, tertiary-buthanol, 2-propanol, ethanol, glycerol, Polyethylene glycol may be provided in the pharmaceutical formulation for lyophilization of a lyophilized product.

Bulking agents and isotonicity makers such as salt e.g. sodium chloride, carbohydrates e.g. dextrose, mannitol, lactose, trehalose, sucrose or mixtures thereof, aminoacids e.g. glycine, glutamate, or excipients such as cystein, lecithin or human serum albumin, or mixtures thereof may be provided in the pharmaceutical composition for lyophilization.

The pharmaceutical compositions of the present invention may be formulated and used as tablets, capsules or elixirs for oral administration; suppositories for rectal administration; preferably sterile solutions or sterile powder or suspensions for injectable administration; and the like. The dose and method of administration can be tailored to achieve optimal efficacy but will depend on such factors as weight, diet, concurrent medication and other factors, which those skilled in the medical arts will recognize.

When administration is to be parenteral, such as intravenous and subcutaneous, e.g., on a daily basis, injectable pharmaceutical compositions can be prepared in conventional forms, either as aqueous solutions or suspensions; lyophilized, solid forms suitable for reconstitution immediately before use or suspension in liquid prior to injection, or as emulsions. It is envisaged that the administration regimen for the peptides of the invention should be peripheral subcutaneous or nasal, e.g. 3 times daily and preprandially.

Diluents for reconstitution of the lyophilized product may be a suitable buffer from the list above, water, saline, dextrose, mannitol, lactose, trehalose, sucrose, lecithin, albumin, sodium glutamate, cysteine hydrochloride; or water for injection with addition of detergents, such as Tween 20, Tween 80, poloxamers e.g. pluronic F-68 or pluronic F-127, polyethylene glycol, and or with addition of preservatives such as para-, meta-, and ortho-cresol, methyl- and propylparaben, phenol, benzyl alcohol, sodium benzoate, benzoic acid, benzyl-benzoate, sorbic acid, propanoic acid, esters of p-hydroxybenzoic acid, and or with addition of an organic modifier such as ethanol, acitic acid, citric acid, lactic acid or salts thereof.

In addition, if desired, the injectable pharmaceutical compositions may contain minor amounts of non-toxic auxiliary substances, such as wetting agents, or pH buffering agents. Absorption enhancing preparations (e.g., liposomes, detergents and organic acids) may be utilized.

In one embodiment of the invention, the compounds are formulated for administration by infusion, e.g., when used as liquid nutritional supplements for patients on total parenteral nutrition therapy (for example neonatals, or patients suffering from cachexia or anorexia), or by injection, for example subcutaneously, intraperitoneal or intravenously, and are accordingly utilized as aqueous solutions in sterile and pyrogen-free form and optionally buffered to physiologically tolerable pH, e.g., a slightly acidic or physiological pH. Formulation for intramuscular administration may be based on solutions or suspensions in plant oil, e.g. canola oil, corn oil or soy bean oil. These oil based formulations may be stabilized by antioxidants e.g. BHA (butylated hydroxianisole) and BHT (butylated hydroxytoluene).

Thus, the present peptide compounds may be administered in a vehicle, such as distilled water or in saline, phosphate buffered saline, 5% dextrose solutions or oils. The solubility of the glucagon analogue may be enhanced, if desired, by incorporating a solubility enhancer, such as detergents and emulsifiers.

The aqueous carrier or vehicle can be supplemented for use as injectables with an amount of gelatin that serves to depot the glucagon analogue at or near the site of injection, for its slow release to the desired site of action. Alternative gelling agents, such as hyaluronic acid, may also be useful as depot agents.

The glucagon analogues of the invention may also be formulated as a slow release implantation device for extended and sustained administration of the glucagon peptide analogue. Such sustained release formulations may be in the form of a patch positioned externally on the body. Examples of sustained release formulations include composites of biocompatible polymers, such as poly(lactic acid), poly(lactic-co-glycolic acid), methylcellulose, hyaluronic acid, sialic acid, silicate, collagen, liposomes and the like. Sustained release formulations may be of particular interest when it is desirable to provide a high local concentration of a glucagon analogue of the invention.

The glucagon analogue may be utilized in the form of a sterile-filled vial or ampoule containing an intestinotrophic amount of the peptide, in either unit dose or multi-dose amounts. The vial or ampoule may contain the glucagon analogue and the desired carrier, as an administration ready formulation. Alternatively, the vial or ampoule may contain the glucagon peptide in a form, such as a lyophilized form, suitable for reconstitution in a suitable carrier, such as sterile water or phosphate-buffered saline.

As an alternative to injectable formulations, the glucagon analogue may be formulated for administration by other routes. Oral dosage forms, such as tablets, capsules and the like, can be formulated in accordance with standard pharmaceutical practice. According to the present invention, the glucagon analogue is administered to treat individuals that would benefit from growth of small bowel tissue.

Nasal dosage forms can be formulated with addition of enhancers, such as Chitosan or detergents such as Tween 20, Tween 80, Poloxamers e.g. Pluronic F-68, Pluronic F-127; Brij 35, Brij 72, cremophor EL.

The peptide compounds of the present invention may be used alone, or in combination with compounds having an anti-inflammatory effect. Without being bound by theory it is envisioned that such combination treatment may enforce the beneficial treatment effects of the present peptide analogues.

The therapeutic dosing and regimen most appropriate for patient treatment will of course vary with the disease or condition to be treated, and according to the patient's weight and other parameters. Without wishing to be bound by any particular theory, it is expected that doses, in the µg/kg range, and shorter or longer duration or frequency of treatment may produce therapeutically useful results, such as a statistically significant increase particularly in small bowel mass. In some instances, the therapeutic regimen may include the administration of maintenance doses appropriate for preventing tissue regression that occurs following cessation of initial treatment. The dosage sizes and dosing regimen most appropriate for human use may be guided by the results obtained by the present invention, and may be confirmed in properly designed clinical trials.

An effective dosage and treatment protocol may be determined by conventional means, starting with a low dose in laboratory animals and then increasing the dosage while monitoring the effects, and systematically varying the dosage regimen as well. Numerous factors may be taken into consideration by a clinician when determining an optimal dosage for a given subject. Such considerations are known to the skilled person.

A human dose of a glucagon peptide according to the invention may in one embodiment be from about 10 µg/kg body weight/day to about 10 mg/kg/day, preferably from about 50 µg/kg/day to about 5 mg/kg/day, and most preferably about 100 µg/kg/day to 1 mg/kg/day.

### Medical Conditions

The peptides of the present invention are useful as a pharmaceutical agent for preventing increasing body weight or promoting loss of body weight. They are therefore useful for treating an individual suffering from excess body weight or obesity, including morbid obesity, metabolic syndrome, type-2 diabetes, hypertension, arteriosclerosis and coronary heart diseases.

It is envisaged that the present peptides may be employed in a method of treating neo-natals by administering an effective amount of a glucagon analogue, or a salt thereof. Complications with feeding neonatals due to the lack of development of the intestine may be overcome by using the present peptide agonists.

### Chemical Stability Testing

Glucagon analogues are dissolved in purified water and subsequently diluted in solutions containing HCl, NaOH, H₂O₂ or NH₄HCO₃. The solutions are incubated at 40°C to produce hydrolysis, deamidation and oxidation products. The samples are analyzed by RP-HPLC and the percentage of remaining intact compound determined as a measure for the relative stability. The major degradation products may be identified by LC-MS. Glucagon is typcally used as a reference for the other glucagon analogues tested.

Chemicals to be used in the experiment are listed in the below.

### Chemicals and reagents used for the analytical procedures.

| **Substance** | **Quality** | **Supplier** | **Product** |
|---|---|---|---|
| Acetonitrile (MeCN) | HPLC grade | Riedel-deHaën | 34851 |
| Trifluoroacetic Acid (TFA) | 99.9 % | Pierce | 28904 |
| Formic Acid (FA) | 98-100% | Merck | 1.00264.1000 |
| Hydrochloric acid HCl | "Baker Analyzed" | J.T.Baker | 7088 |
| Sodium Hydroxide NaOH | "Baker Analyzed" | J.T.Baker | 7098 |
| Hydrogen Peroxide H₂O₂ | 30 % w/w | Sigma-Aldrich | H1009 |
| NH₄HCO₃ | 99.0 % | AnalaR | 103025E |

The water is first de-mineralized to a resistance of ≥ 18 MΩcm and then passed through a Milli-Q system (Millipore, Bedford, USA). The Milli-Q purified water (MQW) is finally tapped through a 0.22-µm sterile filter (Millipak 40 Gamma Gold, Millipore).

The glucagon analogues to be tested and the reference Gly²-glucagon, ZP1559, are dissolved in water and subsequently diluted into solutions containing HCl, NaOH, H₂O₂ or NH₄HCO₃. The samples are incubated at 40°C to generate hydrolysis, deamidation and oxidation products, respectively. The compounds are analysed by RP-HPLC for purity of the original main peak and by LC-MS for confirmation of the identity by mass of the main peak and major degradation products.

### Preparation of stress solutions:

| | |
|---|---|
| 0.2 M HCl: | 4 mL MQW and 1 mL of 1 M HCl. |
| 0.02 M NaOH: | 4 mL MQW and 1 mL of 0.1 M NaOH. |
| 0.2 M NH₄HCO₃, pH 8: | 0.79 g NH₄HCO₃ was dissolved in 50 mL MQW. |
| 1% H₂O₂: | 5.8 mL MQW and 0.2 mL of 30% H₂O₂. |

### Sample solutions:

The glucagon analogues are first dissolved in MQW to a concentration of 4 mg/mL and then further diluted in the stress solutions at a 1:1 ratio (e.g. 125 µL plus 125 µL) to final concentrations of 2 mg/mL of the glucagon analogues for stress conditions with 0.1 M HCl, 0.01 M NaOH, 0.1 M NH₄HCO₃ and 0.5% H₂O₂, respectively.

The solutions are incubated at 40°C in the dark and then diluted in Eluent A to a concentration of 0.5 mg/mL (addition of 750 µL) prior to analyses by RP-HPLC and by LC-MS.

### Conditions for stress test.

| **Solution** | **0.1 M HCl** | **0.01M NaOH** | **0.1 M NH₄HCO₃** | **0.5% H₂O₂** |
|---|---|---|---|---|
| Temperature (°C) | 40 | 40 | 40 | 40 |
| Storage (days) | 12 | 3 | 6 | 3 |

### RP-HPLC

RP-HPLC analyses are performed on an Agilent Series 1100 HPLC system under control of the ChemStation (Revision A.08.03 [847]) software from Agilent Technologies, Inc. The raw data and the results of the peak integration are deposited on the ChemStore C/S server by the use of Agilent Technologies Revision B01.03 software.

### RP-HPLC method.

| | |
|---|---|
| Method file name | P2204071.M |
| Column | Vydac 218MS52, 5 µm, 300 A, 2.1 x 250 mm |
| Gradient (time; % B) | 0;5, 2;5, 7;15, 25;30, 45;40, 65;50, 70;100, 73;100, 75;5, 90;5 |
| Eluent A | 0.05% TFA, 0.05% FA in MQW |
| Eluent B | 0.05% TFA, 0.05% FA in MeCN |
| Flow Rate | 0.200 mL/min |
| Injection Volume | 20 µL |
| Column Temperature | 25°C |
| Auto Sampler Temp. | 4°C |
| UV detection | 220 nm |

### LC-MS

Analytical LC-MS analyses are performed on an Agilent Technologies 1100 HPLC instrument consisting of an on-line degasser, quaternary gradient pump, an auto sampler, a column oven, an UV detector. The HPLC instrument is interfaced with a Micromass LCT (ESI-TOF) mass spectrometer under control of Masslynx 3.5 software, from MicroMass, UK.

### LC-MS method

| | |
|---|---|
| Method file name | P22_04_071_003.M |
| Column | Vydac 218MS52, 5 µm, 300 A, 2.1 x 250 mm |
| Gradient (time; % B) | 0;5, 2;5, 7;15, 25;30, 45;40, 65;50, 70;100, 73;100, 75;5, 90;5 |
| Eluent A | 0.05% TFA, 0.05% FA in MQW |
| Eluent B | 0.05% TFA, 0.05% FA in MeCN |
| Flow Rate | 0.200 mL/min |
| Injection Volume | 30 µL |
| Column Temperature | 25°C |
| Auto Sampler Temp. | 4°C |
| UV detection | 220 nm |

### MS set-up according to SOP 22-3003.

| | |
|---|---|
| Cone voltage | 30 V |
| Capillary voltage | 3.1 kV |
| Nitrogen nebuliser gas flow | 100 L/hr |
| Desolvation gas flow | 500 L/hr |
| Desolvation temperature | 250°C |
| Source block temperature | 100°C |

### Binding assay

Membranes prepared from cells, which express either the hGlucagon-R or hGLP-1-R will be incubated with 30 - 100 pM [125I]GLP-1, [125I]glucagon [125I]PYY in the absence or presence of increasing concentrations (10-12 - 10-6 M) of test peptides in 100 µl binding buffer (25 mM HEPES, 2.5 mM CaCl2, 1 mM MgCl2, & 0.1% BSA, pH 7.4). Non-specific binding will be defined at 1 µM Glucagon or GLP-1. The assay mixtures will be incubated for 30 min at 37°C followed by rapid filtration on Unifilters (GF/C) that have been presoaked in 0.5% polyethylenimin for at least 120 min before use. The filters will be washed 3 times with buffer, dried for 90 min at 60° C, and counted in a TopCount scintillation counter in the presence of scintillation cocktail. IC50 - values will be estimated by computer aided curve fitting.

### Generation of cell lines expressing human glucagon- and GLP-1 receptors

The cDNA encoding either the human glucagon receptor (Glucagon-R) (primary accession number P47871) or the human glucagon-like peptide 1 receptor (GLP-1 R) (primary accession number P43220) were cloned from the cDNA clones BC104854 (MGC:132514/IMAGE:8143857) or BC112126 (MGC:138331/IMAGE:8327594), respectively. The DNA encoding the Glucagon-R or the GLP-1-R was amplified by PCR using primers encoding terminal restriction sites for subcloning. The 5'-end primers additionally encoded a near Kozak consensus sequence to ensure efficient translation. The PCR products encoding the Glucagon-R or the GLP-1-R were subcloned into a mammalian expression vector. The fidelity of the DNA encoding the Glucagon-R and the GLP-1-R was confirmed by DNA sequencing. As the mammalian expression vector additionally encodes for G418 resistance, transfected cells may be selected by applying selection pressure with G418. Cells exhibiting G418 resistance will most likely also express the Glucagon-R or the GLP-1-R. The mammalian expression vectors encoding the Glucagon-R or the GLP-1-R were transfected into HEK293 cells by a standard calcium phosphate transfection method. 48 hr after transfection cells were seeded for limited dilution cloning and selected with 1 mg/ml G418 in the culture medium. Three weeks after 12 surviving colonies of Glucagon-R and GLP-1-R expressing cells were picked, propagated and tested in the Glucagon-R and GLP-1-R efficacy assays as described below. One Glucagon-R expressing clone and one GLP-1-R expressing clone were chosen for compound profiling.

### Glucagon receptor and GLP-1-receptor efficacy assays

HEK293 cells expressing the hGlucagon-R, or hGLP-1-R are seeded at 40,000 cells per well in 96-well microtiter plates coated with 0.01 % poly-L-lysine and grown for 1 day in culture in 100 µM growth medium. On the day of analysis, growth medium is removed and the cells washed once with 200 µl Tyrode buffer. Cells are incubated in 100 µl Tyrode buffer containing increasing concentrations of test peptides, 100 µM IBMX, and 6 mM glucose for up 15 min at 37° C. The reaction is stopped by addition of 25 µl 0.5 M HCl and incubated on ice for 60 min. The cAMP content is estimated using the FlashPlate® cAMP kit from Perkin-Elmer. EC₅₀ and relative efficacy are estimated by computer aided curve fitting.

### We tested the peptide:

H-HSQGTFTSDYSKYLDRARADDFVAWLKST-NH₂ (where the initial H is a hydrogen)
and obtained EC values: GluR: 7.3 pM (SD 1.9) and GLP1R: 9.3pM (SD 1.5) giving a ratio of 1.27.

The corresponding values obtained for human glucagon were GluR: 0.0905 nM and GLP1 R: 2.5 nM giving a ratio of 27.62.

The corresponding values obtained for human oxyntomodulin were GluR: 0.7759 nM and GLP1 R: 2.400 nM (SD 1.5) giving a ratio of 3.09.

### Lipogenesis assay

Hepatocytes are isolated from male Spague Dawley rats (200-300 g) by two-step perfusion technique (Seglen, PO: Preparation of Isolated Rat Liver Cells. Methods Cell Biol. 13: 29-83, 1976). In brief, the liver is initially perfused for 15 minutes with 30 ml/min Ca²⁺-free Hank's Balanced Salt Solution (HBSS) supplemented with 10 mM Hepes and heated to 37°C, followed by perfusion under similar conditions with HBSS supplemented with 10 mM Hepes, 5 mM CaCl₂ and 0.6 mg/ml collagenase type 1 (Worthington Biochemical Corporation, Lakewood, NJ). The liver is subsequently removed, and the hepatocytes liberated into Williams E medium supplemented with 10% fetal calf serum (FCS) and 50 µg/ml gentamycin. Following low-speed centrifugation and washing of the hepatocyte suspension, viable hepatocytes are purified by low-speed, percoll centrifugation (Kreamer BL, Staecker JL, Sawada N, Sattler GL, Hsia MT, Pitot HC: Use of a low-speed, iso-density percoll centrifugation method to increase the viability of isolated rat hepatocyte preparations. In Vitro Cell Dev Biol 22:201-211, 1986). The enriched hepatocyte suspension is washed and filtrated through 100 µm and 70 µm filters. Cell viability, determined by trypan blue exclusion, should be consistently between 85-95%. Cells are plated onto collagen-I-coated 96-well scintiplates TC (Perkin Elmer) at a density of 40,000 cells per well in Williams E medium supplemented with 10% FCS, 50 µg/ml gentamycin, 10 nM insulin and 100 nM dexamethasone. After three hours of culturing at 37°C, 5% CO₂ and the following day, the media was changed to basal medium (Williams E medium supplemented with 0.1% bovine serum albumin and 50 µg/ml gentamycin) containing 10 nM insulin and 100 nM dexamethasone.

Two days after plating, the hepatocytes are washed in basal medium and stimulated for 4 hours at 37°C and 5% CO₂ with different concentrations of test peptides diluted in basal medium supplemented with 2 µCi/ml [1-¹⁴C] sodium acetate (Perkin Elmer). The cells are subsequently washed twice in PBS, the liquid removed, and radioactivity incorporated into cells counted using TopCount NXT Microplate Scintillation Counter.

### Effect of glucagon/GLP-1 co-agonists on food intake and body weight in Diet Induced Obese (DIO) mice

Efficacious glucagon/GLP-1 receptor agonists are tested on 9 - 15 weeks old, 40 C57BI/6J male mice which have been kept on a High Fat Diet (HFD) - 60% high-fat food (Cat. No. D12492: 20% protein, 20% carbohydrate, and 60% fat, 5.2 kcal/g; Research Diets, New Jersey, USA, for 4 weeks. The high-fat food contains soybean oil (25/773.85 g) and lard (245/773.85 g).

DIO-mice are treated with vehicle or co-agonists for 2 - 4 weeks by bolus administration (s.c.) twice daily. Body weight is monitored daily throughout the experiment. At the end of the experimental period animals are sacrificed, blood collected for analysis of glycated hemoglobin and a range of hormones and peptides involved in regulation of appetite, food intake, body weight, and energy balances. In addition, the epididymal/perigonadal fat pads and retroperitoneal fat (WAT) as well as the intrascapular Brown Adipose Tissue (BAT) are dissected out and weighed.

### EXAMPLES

### Effect of glucagon/GLP-1 co-agonist HSQGTFTSDYSKYLDRARADDFVAWLKST-NH₂ (ZP2495) and control peptides Exendin-4 and oxyntomodulin on body weight in Diet Induced Obese (DIO) mice

ZP2495, Exendin-4 and oxyntomodulin were tested on 11 - 14 weeks old, 10 C57BI/6J male mice which had been kept on a High Fat Diet (HFD) - 60% high-fat food (Cat. No. D12492: 20% protein, 20% carbohydrate, and 60% fat, 5.2 kcal/g; Research Diets, New Jersey, USA, for 4 weeks. The high-fat food contained soybean oil (25/773.85 g) and lard (245/773.85 g). All peptides were manufactured according to solid phase peptide synthesis, e.g. as described in WO 98/11126 and WO 99/46283. The peptides used in the experiment have the following analytical data:

| **Drug Name** | **ZP No.** | **MW (g/mol)** | **Peptide Content** | **Purity** | **Solvent** |
|---|---|---|---|---|---|
| Exendin-4 | ZP24 | 4187 | 88 | 98 | PBS |
| Oxyntomodulin | ZP223 2 | 4450 | 83 | 91 | PBS |
| ZP 2495 | ZP249 5 | 3365,7 | 83.1 | 90 | PBS |

Following an acclimatization treatment with 100 µl vehicle (PBS) once a day for two weeks DIO-mice were treated with vehicle, control peptides or ZP2495 for 4 weeks by bolus administration (s.c.) twice daily. Body weight was monitored daily throughout the experiment. At the end of the experimental period animals were sacrificed, blood collected for analysis of glycosylated hemoglobin and a range of hormones and peptides involved in regulation of appetite, food intake, body weight, and energy balances.

Prior to initiation of drug treatment the animals were randomized into groups of 10 animals each with similar average body weight (BW) and an eye blood sample (1 ml (EDTA)) were obtained from all animals (Week 10 of age). On week 7,8,9 and 10 (week 11, 12, 13 and 14 of age) the mice were treated twice daily s.c. with Exendin-4, oxyntomodulin, ZP2495 or vehicle. One group of animals was maintained on regular chow so as to serve as a non-obese/non-diabetic control group. To make the stress of handling the animals comparable to the other groups these animals were treated with PBS in the same regimen as the tested peptides. Throughout the study, body weights were obtained daily, which were used to calculate dose of the drugs. All solutions to be injected were adjusted to the same pH level (pH 6.5) in PBS. The daily injections took place between 8:00-8:30 a.m. and between 16:00-16:30 p.m. with fresh solutions prepared the same day. Prior to sacrifice, an eye blood sample (1 ml (EDTA)) was obtained. All animals were sacrificed at the end of the experiment by cervical dislocation under anesthesia (Hypnorm^{®}-Dormicum^{®}).

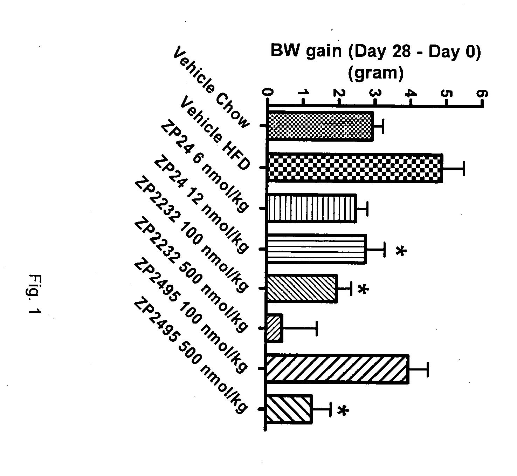

Figure 1 is a column diagram showing body weight gain in high fat fed (DIO) mice treated s.c. with vehicle, Exendin-4 amide (1-39) (ZP24, H-HGEGTFTSDLSKQMEEEAVRLFIEWLKNGGPSSGAPPPS-NH₂), oxyntomodulin (ZP2232, H-HSQGTFTSDYSKYLDSRRAQDFVQWLMNTKRNRNNIA-OH) and the peptide ZP 2495 (H-HSQGTFTSDYSKYLDRARADDFVAWLKST-NH₂) for 28 days (animals eating chow are included for comparison). The figure shows that s.c. bolus administration of 500 nmol/kg twice daily of ZP2495 is able to reduce body weight gain comparable to oxyntomodulin treatment. ( ^{*}p<0.05 compared to vehicle HFD (tukey post-hoc test)).

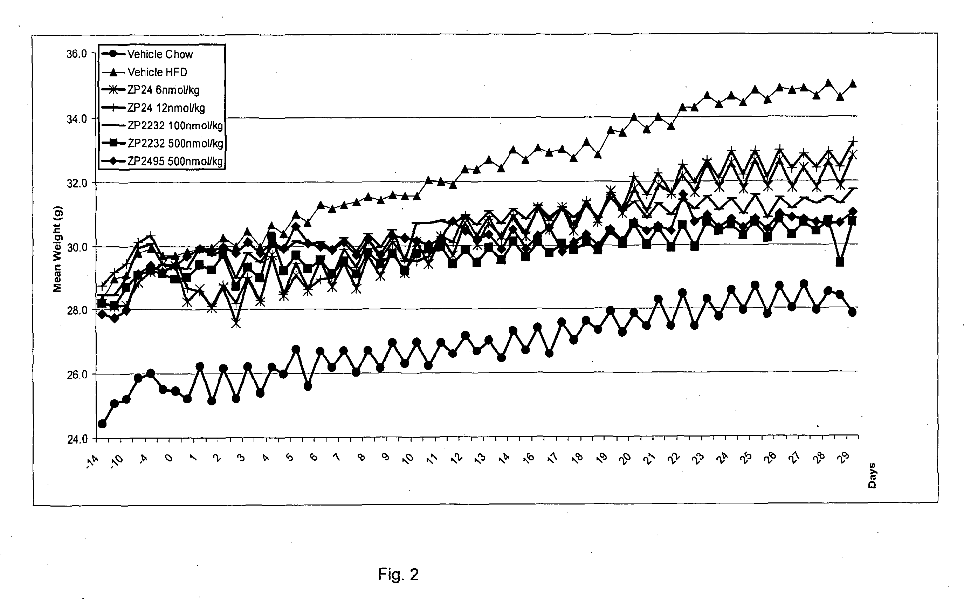

Figure 2 is a chart showing that the weight reducing effect of ZP2495 lin (same control peptides as in Fig. 1) is sustained over time comparable to oxyntomodulin treatment and significantly different from exendin-4 treatment.

While the invention has been described in conjunction with the exemplary embodiments described above, many equivalent modifications and variations will be apparent to those skilled in the art when given this disclosure. Accordingly, the exemplary embodiments of the invention set forth are considered to be illustrative and not limiting. Various changes to the described embodiments may be made without departing from the spirit and scope of the invention. All documents cited herein are expressly incorporated by reference.

## Claims

1. A compound of the formula: 
R¹-Z¹-His-Ser-Gln-Gly-Thr-Phe-Thr-Ser-Asp-Tyr-Ser-X12-Tyr-Leu-Asp-X16-X17-X18-Ala-X20-Asp-
Phe-X23-X24-Trp-Leu-X27-X28-X29-Z²-R²
 wherein:
R¹ is hydrogen, C₁₋₄ alkyl (e.g. methyl), acetyl, formyl, benzoyl or trifluoroacetyl
X12: Lys, Arg, His, Ser, Glu, Asp, Ala;
X16: Ser, Thr, Lys, Arg, His, Glu, Asp, Asn, Ala, Gly;
X17: Arg, Lys, His, Glu, Asp, Gln, Ala, Ser or Leu;
X18: Arg, Lys, His, Glu, Asp, Gln, Tyr, Ala, Ser or Leu;
X20: Gln, Lys, Arg, His, Glu, Asp, Ser, Ala;
X23: Val, Ile, Leu, Ala;
X24: Gln, Lys, Arg, His, Glu, Asp, Ser, Ala or Leu;
X27: Met, Lys, Arg, His, Glu, Asp, Ser, Leu, Ala or absent;
X28: Asn, Lys, Arg, His, Glu, Asp, Ser, Ala or absent;
X29: Thr, Lys, Arg, His, Glu, Asp, Ser, Ala, Gly, Leu or absent;
R² is NH₂ or OH;
Z¹ and Z² are independently absent or a peptide sequence of 1-20 amino acid units selected from the group consisting of Ala, Leu, Ser, Thr, Tyr, Asn, Gln, Asp, Glu, Lys, Arg, His, Met, Har, Dbu, Dpr and Orn wherein, when Z² is present, it has no more than 25% sequence identity to the corresponding sequence of the IP-1 portion of oxyntomodulin;
wherein the glucagon analogue peptide has a substitution relative to human glucagon at one or more of positions 16, 17, 18 or 29, and/or a deletion at one or more of positions 27, 28 and 29;
or a pharmaceutically acceptable salt or derivative thereof;
wherein said glucagon analogue peptide has higher GLP-1 receptor selectivity than human glucagon or wherein said glucagon analogue peptide has greater activity in a lipogenesis assay than human glucagon.

2. A compound of the formula: 
R¹-Z¹-His-Ser-Gln-Gly-Thr-Phe-Thr-Ser-Asp-Tyr-Ser-X12-Tyr-Leu-Asp-X16-X17-X18-Ala-X20-Asp-
Phe-X23-X24-Trp-Leu-X27-X28-X29-Z²-R²
 wherein:
R¹ is hydrogen, C₁₋₄ alkyl (e.g. methyl), acetyl, formyl, benzoyl or trifluoroacetyl
X12: Lys, Arg, His, Ser, Glu, Asp, Ala;
X16: Ser, Thr, Lys, Arg, His, Glu, Asp, Asn, Ala, Gly;
X17: Arg, Lys, His, Glu, Asp, Gln, Ala, Ser or Leu;
X18: Arg, Lys, His, Glu, Asp, Gln, Tyr, Ala, Ser or Leu;
X20: Gln, Lys, Arg, His, Glu, Asp, Ser, Ala;
X23: Val, Ile, Leu, Ala;
X24: Gln, Lys, Arg, His, Glu, Asp, Ser, Ala or Leu;
X27: Met, Lys, Arg, His, Glu, Asp, Ser, Leu, Ala or absent;
X28: Asn, Lys, Arg, His, Glu, Asp, Ser, Ala or absent;
X29: Thr, Lys, Arg, His, Glu, Asp, Ser, Ala, Gly, Leu or absent; ..
R² is NH₂ or OH;
Z¹ and Z² are independently absent or a peptide sequence of 1-20 amino acid units selected from the group consisting of Ala, Leu, Ser, Thr, Tyr, Asn, Gln, Asp, Glu, Lys, Arg, His, Met, Har, Dbu, Dpr and Orn wherein, when Z² is present, it has no more than 25% sequence identity to the corresponding sequence of the IP-1 portion of oxyntomodulin;
wherein the glucagon analogue peptide has a substitution other than Ala at one or more of positions 23, 27 or 28 relative to human glucagon;
or a pharmaceutically acceptable salt or derivative thereof;
wherein said glucagon analogue peptide has higher GLP-1 receptor selectivity than human glucagon, or wherein said glucagon analogue peptide has greater activity in a lipogenesis assay than human glucagon.

3. A compound according to claim 1 or claim 2 further comprising a substitution relative to human glucagon at position 12 and/or 20.

4. A compound according to claim 3 having the residue Arg, Glu or Ser at position 12.

5. A compound according to claim 3 or claim 4 having the residue Lys, Glu or Ser at position 20.

6. A compound according to any one of the preceding claims comprising a deletion at positions 28 and 29.

7. A compound according to claim 6 further comprising a deletion at position 27.

8. A compound according to any one of claims 1 to 5 having substitutions relative to human glucagon at positions 12 and 20, positions 12 and 24, or positions 12 and 29.

9. A compound according to claim 8 having the residues Arg12/Lys20, Arg12/Lys24, or Arg12/Lys29.

10. A compound according to any one of the preceding claims having the residue Leu27.

11. A compound according to claim 1 or claim 2 having the formula:
R¹-Z¹-HSQGTFTSDYSRYLDRRRADDFVAWLLST -Z²-R²;
R¹-Z¹-HSQGTFTSDYSKYLDRARADDFVAWLKST -Z²-R²;
R¹-Z¹-HSQGTFTSDYSKYLDRKVAEEFVAWLKKT-Z²-R²;
R¹-Z¹-HSQGTFTSDYSKYLDRRHADDFVAWLKST-Z²-R²;
R¹-Z¹-HSQGTFTSDYSKYLDERAAKEFVAWLKST-Z²-R²;
R¹-Z¹-HSQGTFTSDYSKYLDERAAEEFVAWLKST-Z²-R²;
R¹-Z¹-HSQGTFTSDYSKYLDEKRAEEFVAWLKST-Z²-R²;
R¹-Z¹-HSQGTFTSDYSKYLDEKRVEEFVAWLKST-Z²-R²;
R¹-Z¹-HSQGTFTSDYSKYLDERAAKEFVEWLKST-Z²-R²;
R¹-Z¹-HSQGTFTSDYSKYLDERAAKEFVKWLKST-Z²-R²;
R¹-Z¹-HSQGTFTSDYSKYLDERAAREFVAWLKST-Z²-R²;
R¹-Z¹-HSQGTFTSDYSKYLDRARAEEFVAWLKST-Z²-R²;
R¹-Z¹-HSOGTFTSDYSKYLDHARAAEFVAWLKAA-Z²-R²;
R¹-Z¹-HSQGTFTSDYSKYLDERRADDFVAWLKST-Z²-R²;
R¹-Z¹-HSQGTFTSDYSKYLDERAAEEFVKWLKST-Z²-R²;
R¹-Z¹-HSQGTFTSDYSKYLDEKKAEEFVAWLKST-Z²-R²; or
R¹-Z¹-HSQGTFTSDYSKYLDERRVEEFVAWLKST-Z²-R²
or a pharmaceutically acceptable salt or derivative thereof.

12. A compound according to any one of the preceding claims, wherein Z¹ is Lys₃, Lys₄, Lys₅, Lys₆ or Lys₇.

13. A compound according to claim 12 wherein R¹ is H.

14. A compound according to any one of the preceding claims, wherein Z² is Lys₃, Lys₄, Lys₅, Lys₆ or Lys₇.

15. A compound according to claim 14 wherein R² is OH.

16. A nucleic acid encoding a compound according to any one of the preceding claims.

17. An expression vector comprising a nucleic acid according to claim 16.

18. A host cell comprising a nucleic acid according to claim 16 or an expression vector according to claim 17.

19. A pharmaceutical composition comprising a compound according to any one of claims 1 to 16, in admixture with pharmaceutically acceptable carrier.

20. Use of a compound, nucleic acid, expression vector or host cell according to any one of claims 1 to 18 in the preparation of a medicament for preventing weight gain or promoting weight loss.

21. A compound, nucleic acid, expression vector or host cell according to any one of claims 1 to 18 for use in a method of medical treatment.


SEQUENCE LISTING

<110>  Zealand Pharma A/S

<120>  Glucagon Analogues

<130>  GRF/FP6488829

<140>  EP 07016032.0
<141>  2007-08-15

<160>  57

<170>  PatentIn version 3.3

<210>  1
<211>  29
<212>  PRT
<213>  Homo sapiens

<400>  1

His Ser Gln Gly Thr Phe Thr Ser Asp Tyr Ser Lys Tyr Leu Asp Ser
1               5                   10                  15

Arg Arg Ala Gln Asp Phe Val Gln Trp Leu Met Asn Thr
            20                  25

<210>  2
<211>  37
<212>  PRT
<213>  Homo sapiens

<400>  2

His Ser Gln Gly Thr Phe Thr Ser Asp Tyr Ser Lys Tyr Leu Asp Ser
1               5                   10                  15

Arg Arg Ala Gln Asp Phe Val Gln Trp Leu Met Asn Thr Lys Arg Asn
            20                  25                  30

Arg Asn Asn Ile Ala
        35

<210>  3
<211>  8
<212>  PRT
<213>  Homo sapiens

<400>  3

Lys Arg Asn Arg Asn Asn Ile Ala
1               5

<210>  4
<211>  29
<212>  PRT
<213>  Artificial sequence

<220>
<221> source
<222> 1..29
<223>  /note= "Description of artificial sequence:  Glucagon analogue
               peptide"

<400>  4

His Ser Gln Gly Thr Phe Thr Ser Asp Tyr Ser Arg Tyr Leu Asp Ser
1               5                   10                  15

Arg Arg Ala Gln Asp Phe Val Gln Trp Leu Met Asn Thr
            20                  25

<210>  5
<211>  29
<212>  PRT
<213>  Artificial sequence

<220>
<221> source
<222> 1..29
<223>  /note= "Description of artificial sequence:  Glucagon analogue
              peptide"

<400>  5

His Ser Gln Gly Thr Phe Thr Ser Asp Tyr Ser Arg Tyr Leu Asp Ser
1               5                   10                  15

Arg Arg Ala Lys Asp Phe Val Gln Trp Leu Met Asn Thr
            20                  25

<210>  6
<211>  29
<212>  PRT
<213>  Artificial sequence

<220>
<221> source
<222> 1..29
<223>  /note= "Description of artificial sequence:  Glucagon analogue
              peptide"

<400>  6

His Ser Gln Gly Thr Phe Thr Ser Asp Tyr Ser Arg Tyr Leu Asp Ser
1               5                   10                  15

Arg Arg Ala Gln Asp Phe Val Lys Trp Leu Met Asn Thr
            20                  25

<210>  7
<211>  29
<212>  PRT
<213>  Artificial sequence

<220>
<221> source
<222> 1..29
<223>  /note= "Description of artificial sequence:  Glucagon analogue
              peptide"

<400>  7

His Ser Gln Gly Thr Phe Thr Ser Asp Tyr Ser Arg Tyr Leu Asp Ser
1               5                   10                  15

Arg Arg Ala Gln Asp Phe Val Gln Trp Leu Met Asn Lys
            20                  25

<210>  8
<211>  29
<212>  PRT
<213>  Artificial sequence

<220>
<221> source
<222> 1..29
<223>  /note= "Description of artificial sequence:  Glucagon analogue
              peptide"

<400>  8

His Ser Gln Gly Thr Phe Thr Ser Asp Tyr Ser Arg Tyr Leu Asp Ser
1               5                   10                  15

Arg Arg Ala Gln Asp Phe Val Gln Trp Leu Leu Asn Thr
            20                  25

<210>  9
<211>  29
<212>  PRT
<213>  Artificial sequence

<220>
<221> source
<222> 1..29
<223>  /note= "Description of artificial sequence:  Glucagon analogue
               peptide"

<400>  9

His Ser Gln Gly Thr Phe Thr Ser Asp Tyr Ser Arg Tyr Leu Asp Ser
1               5                   10                  15

Arg Arg Ala Lys Asp Phe Val Gln Trp Leu Leu Asn Thr
            20                  25

<210>  10
<211>  29
<212>  PRT
<213>  Artificial sequence

<220>
<221> source
<222> 1..29
<223>  /note= "Description of artificial sequence:  Glucagon analogue
               peptide"

<400>  10

His Ser Gln Gly Thr Phe Thr Ser Asp Tyr Ser Arg Tyr Leu Asp Ser
1               5                   10                  15

Arg Arg Ala Gln Asp Phe Val Lys Trp Leu Leu Asn Thr
            20                  25

<210>  11
<211>  29
<212>  PRT
<213>  Artificial sequence

<220>
<221> source
<222> 1..29
<223>  /note= "Description of artificial sequence:  Glucagon analogue
              peptide"

<400>  11

His Ser Gln Gly Thr Phe Thr Ser Asp Tyr Ser Arg Tyr Leu Asp Ser
1               5                   10                  15

Arg Arg Ala Gln Asp Phe Val Gln Trp Leu Leu Asn Lys
            20                  25

<210>  12
<211>  29
<212>  PRT
<213>  Artificial sequence

<220>
<221> source
<222> 1..29
<223>  /note= "Description of artificial sequence:  Glucagon analogue
              peptide"

<400>  12

His Ser Gln Gly Thr Phe Thr Ser Asp Tyr Ser Arg Tyr Leu Asp Arg
1               5                   10                  15

Arg Arg Ala Asp Asp Phe Val Ala Trp Leu Leu Ser Thr
            20                  25

<210>  13
<211>  29
<212>  PRT
<213>  Artificial sequence

<220>
<221> source
<222> 1..29
<223>  /note= "Description of artificial sequence:  Glucagon analogue
              peptide"

<400>  13

His Ser Gln Gly Thr Phe Thr Ser Asp Tyr Ser Lys Tyr Leu Asp Arg
1               5                   10                  15

Ala Arg Ala Asp Asp Phe Val Ala Trp Leu Lys Ser Thr
            20                  25

<210>  14
<211>  29
<212>  PRT
<213>  Artificial sequence

<220>
<221> source
<222> 1..29
<223>  /note= "Description of artificial sequence:  Glucagon analogue
              peptide"

<400>  14

His Ser Gln Gly Thr Phe Thr Ser Asp Tyr Ser Lys Tyr Leu Asp Arg
1               5                   10                  15

Lys Val Ala Glu Glu Phe Val Ala Trp Leu Lys Lys Thr
            20                  25

<210>  15

<211>  29

<212>  PRT

<213>  Artificial sequence

<220>

<221> source

<222> 1..29

<223>  /note= "Description of artificial sequence:  Glucagon analogue
              peptide"

<400>  15

His Ser Gln Gly Thr Phe Thr Ser Asp Tyr Ser Lys Tyr Leu Asp Arg
1               5                   10                  15

Arg His Ala Asp Asp Phe Val Ala Trp Leu Lys Ser Thr
            20                  25

<210>  16

<211>  29

<212>  PRT

<213>  Artificial sequence

<220>

<221> source

<222> 1..29

<223>  /note= "Description of artificial sequence:  Glucagon analogue
              peptide"

<400>  16

His Ser Gln Gly Thr Phe Thr Ser Asp Tyr Ser Lys Tyr Leu Asp Glu
1               5                   10                  15

Arg Ala Ala Lys Glu Phe Val Ala Trp Leu Lys Ser Thr
            20                  25

<210>  17
<211>  29
<212>  PRT
<213>  Artificial sequence

<220>
<221> source
<222> 1..29
<223>  /note= "Description of artificial sequence:  Glucagon analogue
              peptide"

<400>  17

His Ser Gln Gly Thr Phe Thr Ser Asp Tyr Ser Lys Tyr Leu Asp Glu
1               5                   10                  15

Arg Ala Ala Glu Glu Phe Val Ala Trp Leu Lys Ser Thr
            20                  25

<210>  18
<211>  29
<212>  PRT
<213>  Artificial sequence

<220>
<221> source
<222> 1..29
<223>  /note= "Description of artificial sequence:  Glucagon analogue
              peptide"

<400>  18

His Ser Gln Gly Thr Phe Thr Ser Asp Tyr Ser Lys Tyr Leu Asp Glu
1               5                   10                  15

Lys Arg Ala Glu Glu Phe Val Ala Trp Leu Lys Ser Thr
            20                  25

<210>  19
<211>  29
<212>  PRT
<213>  Artificial sequence

<220>
<221> source
<222> 1..29
<223>  /note= "Description of artificial sequence:  Glucagon analogue
              peptide"

<400>  19

His Ser Gln Gly Thr Phe Thr Ser Asp Tyr Ser Lys Tyr Leu Asp Glu
1               5                   10                  15

Lys Arg Val Glu Glu Phe Val Ala Trp Leu Lys Ser Thr
            20                  25

<210>  20
<211>  29
<212>  PRT
<213>  Artificial sequence

<220>
<221> source
<222> 1..29
<223>  /note= "Description of artificial sequence:  Glucagon analogue
              peptide"

<400>  20

His Ser Gln Gly Thr Phe Thr Ser Asp Tyr Ser Lys Tyr Leu Asp Glu
1               5                   10                  15

Arg Ala Ala Lys Glu Phe Val Glu Trp Leu Lys Ser Thr
            20                  25

<210>  21
<211>  29
<212>  PRT
<213>  Artificial sequence

<220>
<221> source
<222> 1..29
<223>  /note= "Description of artificial sequence:  Glucagon analogue
              peptide"

<400>  21

His Ser Gln Gly Thr Phe Thr Ser Asp Tyr Ser Lys Tyr Leu Asp Glu
1               5                   10                  15

Arg Ala Ala Lys Glu Phe Val Lys Trp Leu Lys Ser Thr
            20                  25

<210>  22
<211>  29
<212>  PRT
<213>  Artificial sequence

<220>
<221> source
<222> 1..29
<223>  /note= "Description of artificial sequence:  Glucagon analogue
              peptide"

<400>  22

His Ser Gln Gly Thr Phe Thr Ser Asp Tyr Ser Lys Tyr Leu Asp Glu
1               5                   10                  15

Arg Ala Ala Arg Glu Phe Val Ala Trp Leu Lys Ser Thr
            20                  25

<210>  23
<211>  29
<212>  PRT
<213>  Artificial sequence

<220>
<221> source
<222> 1..29
<223>  /note= "Description of artificial sequence:  Glucagon analogue
              peptide"

<400>  23

His Ser Gln Gly Thr Phe Thr Ser Asp Tyr Ser Lys Tyr Leu Asp Arg
1               5                   10                  15

Ala Arg Ala Glu Glu Phe Val Ala Trp Leu Lys Ser Thr
            20                  25

<210>  24
<211>  29
<212>  PRT
<213>  Artificial sequence

<220>
<221> source
<222> 1..29
<223>  /note= "Description of artificial sequence:  Glucagon analogue
              peptide"

<400>  24

His Ser Gln Gly Thr Phe Thr Ser Asp Tyr Ser Lys Tyr Leu Asp His
1               5                   10                  15

Ala Arg Ala Ala Glu Phe Val Ala Trp Leu Lys Ala Ala
            20                  25

<210>  25
<211>  29
<212>  PRT
<213>  Artificial sequence

<220>
<221> source
<222> 1..29
<223>  /note= "Description of artificial sequence:  Glucagon analogue
            peptide"

<400>  25

His Ser Gln Gly Thr Phe Thr Ser Asp Tyr Ser Lys Tyr Leu Asp Glu
1               5                   10                  15

Arg Arg Ala Asp Asp Phe Val Ala Trp Leu Lys Ser Thr
            20                  25

<210>  26
<211>  29
<212>  PRT
<213>  Artificial sequence

<220>
<221> source
<222> 1..29
<223>  /note= "Description of artificial sequence:  Glucagon analogue
            peptide"

<400>  26

His Ser Gln Gly Thr Phe Thr Ser Asp Tyr Ser Lys Tyr Leu Asp Glu
1               5                   10                  15

Arg Ala Ala Glu Glu Phe Val Lys Trp Leu Lys Ser Thr
            20                  25

<210>  27
<211>  29
<212>  PRT
<213>  Artificial sequence

<220>
<221> source
<222> 1..29
<223>  /note= "Description of artificial sequence:  Glucagon analogue
              peptide"

<400>  27

His Ser Gln Gly Thr Phe Thr Ser Asp Tyr Ser Lys Tyr Leu Asp Glu
1               5                   10                  15

Lys Lys Ala Glu Glu Phe Val Ala Trp Leu Lys Ser Thr
            20                  25

<210>  28
<211>  29
<212>  PRT
<213>  Artificial sequence

<220>
<221> source
<222> 1..29
<223>  /note= "Description of artificial sequence:  Glucagon analogue
              peptide"

<400>  28

His Ser Gln Gly Thr Phe Thr Ser Asp Tyr Ser Lys Tyr Leu Asp Glu
1               5                   10                  15

Arg Arg Val Glu Glu Phe Val Ala Trp Leu Lys Ser Thr
            20                  25

<210>  29
<211>  69
<212>  PRT
<213>  Artificial sequence

<220>
<221> source
<222> 1..69
<223>  /note= "Description of artificial sequence:  Glucagon analogue
            peptide"

<220>
<221>  VARIANT
<222>  (1)..(20)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<220>
<221>  VARIANT
<222>  (32)..(32)
<223>  Xaa is Lys, Arg, His, Ser, Glu, Asp, or Ala

<220>
<221>  VARIANT
<222>  (36)..(36)
<223>  Xaa is Ser, Thr, Lys, Arg, His, Glu, Asp, Asn, Ala or Gly

<220>
<221>  VARIANT
<222>  (37)..(37)
<223>  Xaa is Arg, Lys, His, Glu, Asp, Gln, Ala, Ser or Leu

<220>
<221>  VARIANT
<222>  (38)..(38)
<223>  Xaa is Arg, Lys, His, Glu, Asp, Gln, Tyr, Ala, Ser or Leu

<220>

<221> VARIANT

<222> (40)..(40)

<223> Xaa is Gln, Lys, Arg, His, Glu, Asp, Ser or Ala

<220>

<221> VARIANT

<222> (43)..(43)

<223> Xaa is Val, Ile, Leu or Ala

<220>

<221> VARIANT

<222> (44)..(44)

<223> Xaa is Gln, Lys, Arg, His, Glu, Asp, Ser, Ala or Leu

<220>

<221> VARIANT

<222> (47)..(47)

<223> Xaa is Met, Lys, Arg, His, Glu, Asp, Ser, Leu, Ala or absent

<220>

<221> VARIANT

<222> (48)..(48)

<223> Xaa is Asn, Lys, Arg, His, Glu, Asp, Ser, Ala or absent

<220>

<221> VARIANT

<222> (49)..(49)

<223> Xaa is Thr, Lys, Arg, His, Glu, Asp, Ser, Ala, Gly, Leu or absent

<220>

<221> VARIANT

<222> (50)..(69)

<223> May be present or absent. If present, represents a peptide
sequence of 1 - 20 amino acids wherein each Xaa may be absent or
is selected from the group consisting of Ala, Leu, Ser, Thr, Tyr,
Asn, Gln, Asp, Glu, Lys, Arg, His, Met, Har, Dbu, Dpr & Orn

<400>  29

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Xaa
            20                  25                  30

Tyr Leu Asp Xaa Xaa Xaa Ala Xaa Asp Phe Xaa Xaa Trp Leu Xaa Xaa
        35                  40                  45

Xaa Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210>  30
<211>  69
<212>  PRT
<213>  Artificial sequence

<220>
<221> source
<222> 1..69
<223>  /note= "Description of artificial sequence:  Glucagon analogue
              peptide"

<220>
<221>  VARIANT
<222>  (1)..(20)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<220>
<221> VARIANT
<222> (50)..(69)
<223> May be present or absent. If present, represents a peptide
      sequence of 1 - 20 amino acids wherein each Xaa may be absent or
      is selected from the group consisting of Ala, Leu, Ser, Thr, Tyr,
      Asn, Gln, Asp, Glu, Lys, Arg, His, Met, Har, Dbu, Dpr & Orn

<400> 30

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Arg
            20                  25                  30

Tyr Leu Asp Arg Arg Arg Ala Asp Asp Phe Val Ala Trp Leu Leu Ser
        35                  40                  45

Thr Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210> 31
<211> 69
<212> PRT
<213> Artificial sequence

<220>
<221> source
<222> 1..69
<223> /note= "Description of artificial sequence: Glucagon analogue
          peptide"

<220>
<221> VARIANT
<222> (1)..(20)
<223> May be present or absent. If present, represents a peptide
      sequence of 1 - 20 amino acids wherein each Xaa may be absent or
      is selected from the group consisting of Ala, Leu, Ser, Thr, Tyr,
      Asn, Gln, Asp, Glu, Lys, Arg, His, Met, Har, Dbu, Dpr & Orn

<220>
<221> VARIANT
<222> (50)..(69)
<223> May be present or absent. If present, represents a peptide
      sequence of 1 - 20 amino acids wherein each Xaa may be absent or
      is selected from the group consisting of Ala, Leu, Ser, Thr, Tyr,
      Asn, Gln, Asp, Glu, Lys, Arg, His, Met, Har, Dbu, Dpr & Orn

<400> 31

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Lys
            20                  25                  30

Tyr Leu Asp Arg Ala Arg Ala Asp Asp Phe Val Ala Trp Leu Lys Ser
        35                  40                  45

Thr Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210>  32

<211>  69

<212>  PRT

<213>  Artificial sequence

<220>

<221> source

<222> 1..69

<223>  /note= "Description of artificial sequence:  Glucagon analogue
            peptide"

<220>

<221>  VARIANT

<222>  (1)..(20)

<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<220>

<221>  VARIANT

<222>  (50)..(69)

<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<400>  32

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Lys
            20                  25                  30

Tyr Leu Asp Arg Lys Val Ala Glu Glu Phe Val Ala Trp Leu Lys Lys
        35                  40                  45

Thr Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210>  33
<211>  69
<212>  PRT
<213>  Artificial sequence

<220>
<221> source
<222> 1..69
<223>  /note= "Description of artificial sequence:  Glucagon analogue
        peptide"

<220>
<221>  VARIANT
<222>  (1)..(20)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<220>
<221>  VARIANT
<222>  (50)..(69)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<400>  33

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Lys
            20                  25                  30

Tyr Leu Asp Arg Arg His Ala Asp Asp Phe Val Ala Trp Leu Lys Ser
        35                  40                  45

Thr Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210>  34
<211>  69
<212>  PRT
<213>  Artificial sequence

<220>
<221> source
<222> 1..69
<223>  /note= "Description of artificial sequence:  Glucagon analogue
               peptide"

<220>
<221>  VARIANT
<222>  (1)..(20)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<220>

<221> VARIANT

<222> (50)..(69)

<223> May be present or absent. If present, represents a peptide
      sequence of 1 - 20 amino acids wherein each Xaa may be absent or
      is selected from the group consisting of Ala, Leu, Ser, Thr, Tyr,
      Asn, Gln, Asp, Glu, Lys, Arg, His, Met, Har, Dbu, Dpr & Orn

<400> 34

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Lys
            20                  25                  30

Tyr Leu Asp Glu Arg Ala Ala Lys Glu Phe Val Ala Trp Leu Lys Ser
        35                  40                  45

Thr Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210> 35
<211> 69
<212> PRT
<213> Artificial sequence

<220>
<221> source
<222> 1..69
<223> /note= "Description of artificial sequence: Glucagon analogue
          peptide"

<220>
<221> VARIANT
<222> (1)..(20)
<223> May be present or absent. If present, represents a peptide
sequence of 1 - 20 amino acids wherein each Xaa may be absent or
is selected from the group consisting of Ala, Leu, Ser, Thr, Tyr,
Asn, Gln, Asp, Glu, Lys, Arg, His, Met, Har, Dbu, Dpr & Orn

<220>
<221> VARIANT
<222> (50)..(69)
<223> May be present or absent. If present, represents a peptide
sequence of 1 - 20 amino acids wherein each Xaa may be absent or
is selected from the group consisting of Ala, Leu, Ser, Thr, Tyr,
Asn, Gln, Asp, Glu, Lys, Arg, His, Met, Har, Dbu, Dpr & Orn

<400> 35

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Lys
            20                  25                  30

Tyr Leu Asp Glu Arg Ala Ala Glu Glu Phe Val Ala Trp Leu Lys Ser
        35                  40                  45

Thr Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210>  36
<211>  69
<212>  PRT
<213>  Artificial sequence

<220>
<221> source
<222> 1..69
<223>  /note= "Description of artificial sequence:  Glucagon analogue
              peptide"

<220>
<221>  VARIANT
<222>  (1)..(20)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<220>
<221>  VARIANT
<222>  (50)..(69)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<400>  36

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Lys
            20                  25                  30

Tyr Leu Asp Glu Lys Arg Ala Glu Glu Phe Val Ala Trp Leu Lys Ser
        35                  40                  45

Thr Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210>  37
<211>  69
<212>  PRT
<213>  Artificial sequence

<220>
<221> source
<222> 1..69
<223>  /note= "Description of artificial sequence:  Glucagon analogue
               peptide"

<220>
<221>  VARIANT
<222>  (1)..(20)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<220>
<221>  VARIANT
<222>  (50)..(69)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<400>  37

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Lys
            20                  25                  30

Tyr Leu Asp Glu Lys Arg Val Glu Glu Phe Val Ala Trp Leu Lys Ser
        35                  40                  45

Thr Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210>  38
<211>  69
<212>  PRT
<213>  Artificial sequence

<220>
<221> source
<222> 1..69
<223>  /note= "Description of artificial sequence:  Glucagon analogue
              peptide"

<220>
<221>  VARIANT
<222>  (1)..(20)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<220>
<221> VARIANT
<222> (50)..(69)
<223> May be present or absent. If present, represents a peptide
      sequence of 1 - 20 amino acids wherein each Xaa may be absent or
      is selected from the group consisting of Ala, Leu, Ser, Thr, Tyr,
      Asn, Gln, Asp, Glu, Lys, Arg, His, Met, Har, Dbu, Dpr & Orn

<400> 38

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Lys
            20                  25                  30

Tyr Leu Asp Glu Arg Ala Ala Lys Glu Phe Val Glu Trp Leu Lys Ser
        35                  40                  45

Thr Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210> 39
<211> 69
<212> PRT
<213> Artificial sequence

<220>
<221> source
<222> 1..69
<223> /note= "Description of artificial sequence: Glucagon analogue
          peptide"

<220>
<221> VARIANT
<222> (1)..(20)
<223> May be present or absent. If present, represents a peptide
sequence of 1 - 20 amino acids wherein each Xaa may be absent or
is selected from the group consisting of Ala, Leu, Ser, Thr, Tyr,
Asn, Gln, Asp, Glu, Lys, Arg, His, Met, Har, Dbu, Dpr & Orn

<220>
<221> VARIANT
<222> (50)..(69)
<223> May be present or absent. If present, represents a peptide
sequence of 1 - 20 amino acids wherein each Xaa may be absent or
is selected from the group consisting of Ala, Leu, Ser, Thr, Tyr,
Asn, Gln, Asp, Glu, Lys, Arg, His, Met, Har, Dbu, Dpr & Orn

<400> 39

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Lys
            20                  25                  30

Tyr Leu Asp Glu Arg Ala Ala Lys Glu Phe Val Lys Trp Leu Lys Ser
        35                  40                  45

Thr Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210>  40
<211>  69
<212>  PRT
<213>  Artificial sequence

<220>
<221> source
<222> 1..69
<223>  /note= "Description of artificial sequence:  Glucagon analogue
            peptide"

<220>
<221>  VARIANT
<222>  (1)..(20)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<220>
<221>  VARIANT
<222>  (50)..(69)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<400>  40

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Lys
            20                  25                  30

Tyr Leu Asp Glu Arg Ala Ala Arg Glu Phe Val Ala Trp Leu Lys Ser
        35                  40                  45

Thr Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210>  41
<211>  69
<212>  PRT
<213>  Artificial sequence

<220>
<221> source
<222> 1..69
<223>  /note= "Description of artificial sequence:  Glucagon analogue
               peptide"

<220>
<221>  VARIANT
<222>  (1)..(20)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<220>
<221>  VARIANT
<222>  (50)..(69)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<400>  41

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Lys
            20                  25                  30

Tyr Leu Asp Arg Ala Arg Ala Glu Glu Phe Val Ala Trp Leu Lys Ser
        35                  40                  45

Thr Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210> 42
<211> 69
<212> PRT
<213> Artificial sequence

<220>
<221> source
<222> 1..69
<223> /note= "Description of artificial sequence: Glucagon analogue
        peptide"

<220>
<221> VARIANT
<222> (1)..(20)
<223> May be present or absent. If present, represents a peptide
      sequence of 1 - 20 amino acids wherein each Xaa may be absent or
      is selected from the group consisting of Ala, Leu, Ser, Thr, Tyr,
      Asn, Gln, Asp, Glu, Lys, Arg, His, Met, Har, Dbu, Dpr & Orn

<220>
<221> VARIANT
<222> (50)..(69)
<223> May be present or absent. If present, represents a peptide
      sequence of 1 - 20 amino acids wherein each Xaa may be absent or
      is selected from the group consisting of Ala, Leu, Ser, Thr, Tyr,
      Asn, Gln, Asp, Glu, Lys, Arg, His, Met, Har, Dbu, Dpr & Orn

<400> 42

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Lys
            20                  25                  30

Tyr Leu Asp His Ala Arg Ala Ala Glu Phe Val Ala Trp Leu Lys Ala
        35                  40                  45

Ala Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210> 43
<211> 69
<212> PRT
<213> Artificial sequence

<220>
<221> source
<222> 1..69
<223> /note= "Description of artificial sequence: Glucagon analogue
      peptide"

<220>
<221> VARIANT
<222> (1)..(20)
<223> May be present or absent. If present, represents a peptide
      sequence of 1 - 20 amino acids wherein each Xaa may be absent or
      is selected from the group consisting of Ala, Leu, Ser, Thr, Tyr,
      Asn, Gln, Asp, Glu, Lys, Arg, His, Met, Har, Dbu, Dpr & Orn

<220>
<221> VARIANT
<222> (50)..(69)
<223> May be present or absent. If present, represents a peptide
      sequence of 1 - 20 amino acids wherein each Xaa may be absent or
      is selected from the group consisting of Ala, Leu, Ser, Thr, Tyr,
      Asn, Gln, Asp, Glu, Lys, Arg, His, Met, Har, Dbu, Dpr & Orn

<400> 43

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Lys
            20                  25                  30

Tyr Leu Asp Glu Arg Arg Ala Asp Asp Phe Val Ala Trp Leu Lys Ser
        35                  40                  45

Thr Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210>  44

<211>  69

<212>  PRT

<213>  Artificial sequence

<220>

<221> source

<222> 1..69

<223>  /note= "Description of artificial sequence:  Glucagon analogue
        peptide"

<220>

<221>  VARIANT

<222>  (1)..(20)

<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<220>

<221>  VARIANT

<222>  (50)..(69)

<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<400>  44

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Lys
            20                  25                  30

Tyr Leu Asp Glu Arg Ala Ala Glu Glu Phe Val Lys Trp Leu Lys Ser
        35                  40                  45

Thr Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210>  45
<211>  69
<212>  PRT
<213>  Artificial sequence

<220>
<221> source
<222> 1..69
<223>  /note= "Description of artificial sequence:  Glucagon analogue
            peptide"

<220>
<221>  VARIANT
<222>  (1)..(20)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<220>
<221>  VARIANT
<222>  (50)..(69)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<400>  45

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Lys
            20                  25                  30

Tyr Leu Asp Glu Lys Lys Ala Glu Glu Phe Val Ala Trp Leu Lys Ser
        35                  40                  45

Thr Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210>  46
<211>  69
<212>  PRT
<213>  Artificial sequence

<220>
<221> source
<222> 1..69
<223>  /note= "Description of artificial sequence:  Glucagon analogue
               peptide"

<220>
<221>  VARIANT
<222>  (1)..(20)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<220>

<221>  VARIANT

<222>  (50)..(69)

<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<400>  46

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Lys
            20                  25                  30

Tyr Leu Asp Glu Arg Arg Val Glu Glu Phe Val Ala Trp Leu Lys Ser
        35                  40                  45

Thr Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210>  47
<211>  69
<212>  PRT
<213>  Artificial sequence

<220>
<221> source
<222> 1..69
<223>  /note= "Description of artificial sequence:  Glucagon analogue
            peptide"

<220>

<221>  VARIANT

<222>  (1)..(20)

<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<220>

<221>  VARIANT

<222>  (50)..(69)

<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<400>  47

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Arg
            20                  25                  30

Tyr Leu Asp Ser Arg Arg Ala Gln Asp Phe Val Gln Trp Leu Met Asn
        35                  40                  45

Thr Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210>  48
<211>  69
<212>  PRT
<213>  Artificial sequence

<220>
<221> source
<222> 1..69
<223>  /note= "Description of artificial sequence:  Glucagon analogue
          peptide"

<220>
<221>  VARIANT
<222>  (1)..(20)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<220>
<221>  VARIANT
<222>  (50)..(69)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<400>  48

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Arg
            20                  25                  30

Tyr Leu Asp Ser Arg Arg Ala Lys Asp Phe Val Gln Trp Leu Met Asn
        35                  40                  45

Thr Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210>  49
<211>  69
<212>  PRT
<213>  Artificial sequence

<220>
<221> source
<222> 1..69
<223>  /note= "Description of artificial sequence:  Glucagon analogue
        peptide"

<220>
<221>  VARIANT
<222>  (1)..(20)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<220>
<221>  VARIANT
<222>  (50)..(69)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<400>  49

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Arg
            20                  25                  30

Tyr Leu Asp Ser Arg Arg Ala Gln Asp Phe Val Lys Trp Leu Met Asn
        35                  40                  45

Thr Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210>  50
<211>  69
<212>  PRT
<213>  Artificial sequence

<220>
<221> source
<222> 1..69
<223>  /note= "Description of artificial sequence:  Glucagon analogue
        peptide"

<220>
<221>  VARIANT
<222>  (1)..(20)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<220>
<221> VARIANT
<222> (50)..(69)
<223> May be present or absent. If present, represents a peptide
sequence of 1 - 20 amino acids wherein each Xaa may be absent or
is selected from the group consisting of Ala, Leu, Ser, Thr, Tyr,
Asn, Gln, Asp, Glu, Lys, Arg, His, Met, Har, Dbu, Dpr & Orn

<400> 50

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Arg
            20                  25                  30

Tyr Leu Asp Ser Arg Arg Ala Gln Asp Phe Val Gln Trp Leu Met Asn
        35                  40                  45

Lys Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210> 51
<211> 69
<212> PRT
<213> Artificial sequence

<220>
<221> source
<222> 1..69
<223> /note= "Description of artificial sequence: Glucagon analogue
peptide"

<220>
<221>  VARIANT
<222>  (1)..(20)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<220>
<221>  VARIANT
<222>  (50)..(69)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<400>  51

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Arg
            20                  25                  30

Tyr Leu Asp Ser Arg Arg Ala Gln Asp Phe Val Gln Trp Leu Leu Asn
        35                  40                  45

Thr Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210>  52
<211>  69
<212>  PRT
<213>  Artificial sequence

<220>
<221> source
<222> 1..69
<223>  /note= "Description of artificial sequence:  Glucagon analogue
          peptide"

<220>
<221>  VARIANT
<222>  (1)..(20)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<220>
<221>  VARIANT
<222>  (50)..(69)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<400>  52

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Arg
            20                  25                  30

Tyr Leu Asp Ser Arg Arg Ala Lys Asp Phe Val Gln Trp Leu Leu Asn
        35                  40                  45

Thr Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210>  53
<211>  69
<212>  PRT
<213>  Artificial sequence

<220>
<221> source
<222> 1..69
<223>  /note= "Description of artificial sequence:  Glucagon analogue
               peptide"

<220>
<221>  VARIANT
<222>  (1)..(20)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<220>
<221>  VARIANT
<222>  (50)..(69)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<400>  53

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Arg
            20                  25                  30

Tyr Leu Asp Ser Arg Arg Ala Gln Asp Phe Val Lys Trp Leu Leu Asn
        35                  40                  45

Thr Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210> 54
<211> 69
<212> PRT
<213> Artificial sequence

<220>
<221> source
<222> 1..69
<223> /note= "Description of artificial sequence: Glucagon analogue
        peptide"

<220>
<221> VARIANT
<222> (1)..(20)
<223> May be present or absent. If present, represents a peptide
      sequence of 1 - 20 amino acids wherein each Xaa may be absent or
      is selected from the group consisting of Ala, Leu, Ser, Thr, Tyr,
      Asn, Gln, Asp, Glu, Lys, Arg, His, Met, Har, Dbu, Dpr & Orn

<220>
<221> VARIANT
<222> (50)..(69)
<223> May be present or absent. If present, represents a peptide
      sequence of 1 - 20 amino acids wherein each Xaa may be absent or
      is selected from the group consisting of Ala, Leu, Ser, Thr, Tyr,
      Asn, Gln, Asp, Glu, Lys, Arg, His, Met, Har, Dbu, Dpr & Orn

<400> 54

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa His Ser Gln Gly Thr Phe Thr Ser Asp Tyr Ser Arg
            20                  25                  30

Tyr Leu Asp Ser Arg Arg Ala Gln Asp Phe Val Gln Trp Leu Leu Asn
        35                  40                  45

Lys Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa
65

<210> 55
<211> 49
<212> PRT
<213> Artificial sequence

<220>
<221> source
<222> 1..49
<223> /note= "Description of artificial sequence: Glucagon analogue
            peptide"

<220>
<221> VARIANT
<222> (1)..(10)
<223> May be present or absent. If present, represents a peptide
      sequence of 1 - 20 amino acids wherein each Xaa may be absent or
      is selected from the group consisting of Ala, Leu, Ser, Thr, Tyr,
      Asn, Gln, Asp, Glu, Lys, Arg, His, Met, Har, Dbu, Dpr & Orn

<220>
<221> VARIANT
<222> (26)..(26)
<223> Xaa may be Ser, Thr, Lys, Arg or His

<220>
<221> VARIANT
<222> (27)..(27)
<223> Xaa may be Ala, Ser or Leu

<220>
<221> VARIANT
<222> (28)..(28)
<223> Xaa may be Arg, Lys, His, Ala, Ser or Leu

<220>
<221> VARIANT
<222> (30)..(30)
<223> Xaa may be Gln, Lys, Arg, His, Glu, Asp, Ser or Ala

<220>
<221> VARIANT
<222> (33)..(33)
<223> Xaa may be Val, Ile, Leu or Ala

<220>
<221> VARIANT
<222> (34)..(34)
<223> Xaa may be Ser, Ala or Leu

<220>
<221> VARIANT
<222> (37)..(37)
<223> Xaa may be Lys, Arg or His

<220>
<221> VARIANT
<222> (38)..(38)
<223> Xaa may be Ser or Ala

<220>
<221> VARIANT
<222> (39)..(39)
<223> Xaa may be Thr, Lys, Arg, His, Glu, Asp, Ser, Ala, Gly,
Leu or absent

<220>
<221> VARIANT
<222> (40)..(49)
<223> May be present or absent. If present, represents a peptide
sequence of 1 - 20 amino acids wherein each Xaa may be absent or
is selected from the group consisting of Ala, Leu, Ser, Thr, Tyr,
Asn, Gln, Asp, Glu, Lys, Arg, His, Met, Har, Dbu, Dpr & Orn

<400> 55

Xaa Xaa Xaa Xaa Xaa Xaa Xaa Xaa Xaa Xaa His Ser Gln Gly Thr Phe
1               5                   10                  15

Thr Ser Asp Tyr Ser Lys Tyr Leu Asp Xaa Xaa Xaa Ala Xaa Asp Phe
            20                  25                  30

Xaa Xaa Trp Leu Xaa Xaa Xaa Xaa Xaa Xaa Xaa Xaa Xaa Xaa Xaa Xaa
        35                  40                  45

Xaa

<210>  56
<211>  49
<212>  PRT
<213>  Artificial sequence

<220>
<221> source
<222> 1..49
<223>  /note= "Description of artificial sequence:  Glucagon analogue
        peptide"

<220>
<221>  VARIANT
<222>  (1)..(10)
<223>  May be present or absent. If present, represents a peptide
       sequence of 1 - 20 amino acids wherein each Xaa may be absent or
       is selected from the group consisting of Ala, Leu, Ser, Thr, Tyr,
       Asn, Gln, Asp, Glu, Lys, Arg, His, Met, Har, Dbu, Dpr & Orn

<220>
<221>  VARIANT
<222>  (26)..(26)
<223>  Xaa may be Ser, Lys, Arg, His or Glu

<220>
<221>  VARIANT
<222>  (27)..(27)
<223>  Xaa may be Ala, Arg, Lys, Glu or Leu

<220>
<221>  VARIANT
<222>  (28)..(28)
<223>  Xaa may be Arg, Lys, His, Ala, Ser or Leu

<220>
<221>  VARIANT
<222>  (30)..(30)
<223>  Xaa may be Gln, Lys, Arg, His, Glu, Asp, Ser, Ala or Leu

<220>
<221> VARIANT
<222> (33)..(33)
<223> Xaa may be Val, Ile, Leu or Ala

<220>
<221> VARIANT
<222> (34)..(34)
<223> Xaa may be Ala, Leu, Lys, Glu or Ser

<220>
<221> VARIANT
<222> (37)..(37)
<223> Xaa may be Lys, Arg or His

<220>
<221> VARIANT
<222> (38)..(38)
<223> Xaa may be Ser, Lys or Glu

<220>
<221> VARIANT
<222> (39)..(39)
<223> Xaa may be Thr, Lys, Glu, Ser, Ala, Gly, Leu or absent

<220>
<221> VARIANT
<222> (40)..(49)
<223> May be present or absent. If present, represents a peptide
      sequence of 1 - 20 amino acids wherein each Xaa may be absent or
      is selected from the group consisting of Ala, Leu, Ser, Thr, Tyr,
      Asn, Gln, Asp, Glu, Lys, Arg, His, Met, Har, Dbu, Dpr & Orn

<400> 56

Xaa Xaa Xaa Xaa Xaa Xaa Xaa Xaa Xaa Xaa His Ser Gln Gly Thr Phe
1               5                   10                  15

Thr Ser Asp Tyr Ser Lys Tyr Leu Asp Xaa Xaa Xaa Ala Xaa Asp Phe
            20                  25                  30

Xaa Xaa Trp Leu Xaa Xaa Xaa Xaa Xaa Xaa Xaa Xaa Xaa Xaa Xaa Xaa
        35                  40                  45

Xaa

<210>  57
<211>  39
<212>  PRT
<213>  Artificial sequence

<220>
<221> source
<222> 1..39
<223>  /note= "Description of artificial sequence:  Exendin-4 amide"

<400>  57

His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser
        35